(19)

Europäisches
Patentamt
European
Patent Office
Office européen
des brevets

(11) **EP 2 594 295 A1**

(12) **EUROPEAN PATENT APPLICATION**

(43) Date of publication:
**22.05.2013 Bulletin 2013/21**

(21) Application number: **11382349.6**

(22) Date of filing: **16.11.2011**

(51) Int Cl.:
***A61L 27/20*** *(2006.01)*   ***A61L 27/22*** *(2006.01)*
***A61L 27/26*** *(2006.01)*   ***A61L 27/38*** *(2006.01)*
***A61L 27/52*** *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicants:
- **Servicio Andaluz de Salud
  41071 Sevilla (ES)**
- **Universidad De Granada
  18071 Granada (ES)**

(72) Inventors:
- **Garrido Gómez, Juan
  E-41071 Sevilla (ES)**

- **González Andrades, Miguel
  E-41071 Sevilla (ES)**
- **Alaminos Mingorance, Miguel
  E-18071 Granada (ES)**
- **Campos Muñoz, Antonio
  E-18071 Granada (ES)**
- **Carriel Araya, Víctor Sebastián
  E-18071 Granada (ES)**

(74) Representative: **ABG Patentes, S.L.
  Avenida de Burgos, 16D
  Edificio Euromor
  28036 Madrid (ES)**

(54) **Nerve implants based on a compacted biomaterial containing cells**

(57)    The invention relates to artificial tissues which are suitable as biocompatible implants for the surgical repair of transected or damaged nerves. The artificial tissue is a nanostructured tissue obtained by compacting and thereby expelling the interstitial fluid of a matrix comprising fibrin, a polysaccharide and stem cells.

EP 2 594 295 A1

**Description**

**FIELD OF THE INVENTION**

The invention relates to the field of neurology and, more in particular, to the field of biocompatible implants based on nanostructured biomaterials comprising stem cells which are useful for the surgical repair of transected or damaged nerves.

**BACKGROUND OF THE INVENTION**

The peripheral nervous system (PNS) extends outside the central nervous system (CNS) and provides the functions of, amongst other things, bringing sensory information to the CNS and receiving motor commands from the CNS, coordinating body movements and controlling the involuntary muscles. Unlike the central nervous system, the PNS is not protected by bone and is therefore vulnerable to injuries.

Damage to nerves of the PNS can cause significant motor or sensory impairment. In particular, patients with acute peripheral nerve injury usually have nerve conduction defects that can manifest as motor impairment or sensory dysfunction. Depending on the severity of the injury and the nerve affected, an injured nerve may cause paralysis, partial loss of mobility of the affected limb and/or a loss of sensation. Nerve and muscle atrophy will follow if no sufficient recovery occurs or no timely treatment is provided. Similarly, crush damage to peripheral nerves can result in reduced motor or sensory performance.

In the case of peripheral nerve being severed surgically or severed due to injury, an initial attempt is made to directly anastomose the stumps of the severed peripheral nerve. In many cases, however, it is impossible to accurately anastomose the severed nerve directly resulting in the nerve being left in the severed state. Consequently, although the nerve spontaneously attempts to regenerate towards the distal side, it is impaired by connective tissue. Hence, regeneration stops with the formation of a neuroma at the severed end without reaching the neural stump on the distal side. As a result, the function of the severed nerve is frequently not fully restored after the surgical wound or injury has healed, and deficits remain. In cases in which direct anastomosis is not possible, a peripheral nerve having a function which is not very important may be partially excised from the same patient, and autotransplantation may be performed to the severed site of the nerve using this peripheral nerve segment. However, in this method as well, not only are there many cases in which nerve function is not adequately restored, but there are also many cases in which decreased function is observed even at the portion at which the transplanted nerve is used.

In addition to the suturing of the severed ends of the nerve, several techniques have previously been attempted to aid and accelerate the repair of damaged nerves, suturing an allograft or autograft, or regenerating the nerve through a biological or synthetic conduit.

Autografts and allografts require a segment of a donor nerve acquired from the patient (autograft) or a donor (allograft). The donor nerve segment is removed from another part of the body or a donor and then sutured between the unattached ends at the injury site. Nerve autograft procedures are difficult, expensive, and offer limited success. Often, a second surgical procedure is required and may lead to permanent denervation at the nerve donor site. Allografts typically require the use of immunosuppressive drugs to avoid rejection of donor segments.

Therefore, numerous attempts have been made to restore function by connecting the stumps of severed peripheral nerves with a tube-shaped material, namely an artificial tube for nerve, regenerating the axon from the stump on the central side of the nerve trunk towards the stump on the distal side, inducing the nerve to extend in the proper direction, and allowing the nerve to reach a myoneural junction or peripheral sensory receptor from the peripheral nerve trunk. In the past, various materials have been attempted to be used as artificial tube for nerve, examples of which include non-porous tubes made of silicone, polyethylene or polyvinyl chloride, porous tubes made of drawn polytetrafluoroethylene or cellulose, semi-permeable membrane tubes made of polyacrylonitrile or polysulfone, tubes made of biodegradable materials such as polyglycolic acid, polylactic acid or their copolymers, gelatin tubes, or biological tissue tubes originating in the same species such as arteries and veins. However, in regeneration experiments on peripheral nerves using these materials, since biological repair is impaired by the material, the length of nerve that has been able to be regenerated thus far has been at most on the order of 15 mm. In addition, not only is the regenerated nerve narrow without the form of the nerve being normally restored, but there are also many cases in which the function of regenerated nerve is not restored. In addition, although examples have been reported in which neural growth factor NGF is filled into a tube, since NGF ends up rapidly running out of the tube and dispersing, remarkable effects have not been obtained.

Although artificial tubes for nerve regeneration which comprise collagen tubes which coated or filled with laminin and fibronectin have recently been attempted (Tong, X., et al., Brain Research 663: 155-162 (1994), since the collagen tubes are unable to remain without being broken down until the nerve is regenerated to an adequate length, satisfactory results have not yet been obtained with this biomaterial.

US4863668 describes a material used for the regeneration of nerve cells comprising a mandrel coated with several layers of a cross-linked matrix of collagen and fibrin.

Garrido-Gómez et al. (Histology and Histopathology, 2011, 26, suppl. 1, abstract 50.4.o4) have described a method for nerve regeneration which comprises the use of a three-dimensional fibrin-agarose neural construct with adipose tissue-derived stem cells.

WO0154593 describes a method for nerve regeneration using a neural regeneration conduit comprising a support based on small intestinal submucosa and having spiral geometry and which is coated with Schwann cells. However, isolation of autologous Schwann cells still requires nerve tissue harvested from the patient, and therefore, has similar disadvantages as autografts.

Stem cells have been viewed as a solution to the problem of the use of cells of the patients, including both differentiated and undifferentiated states (Tohill M. et al., Biotechnol. Appl. Biochem., 2004; 40: 17-24). While this approach has attracted much attention, particularly for application to the central nervous system, neuronal phenotypes are among the most difficulty to reliably differentiate in high yields from adult stem cells, the most likely source for near-term clinical application to peripheral nerve repair (Kokai L.E. et al., Plast Reconstr Surg 2005;1 16(5): 1453-60; Chen Y. et al., Cell Mol Life Sci 2006;63(14): 1649-57).

However, there is a need in the art for additional nerve regenerating materials which overcomes the disadvantages of the materials known to date.

## SUMMARY OF THE INVENTION

In a first aspect, the invention relates to a method for producing a nanostructured biomaterial capable of regenerating a nerve lesion comprising the steps of:

(i) providing a hydrogel comprising a polysaccharide network, a fibrin network and cells embedded within said hydrogel and

(ii) compacting the biomaterial of step (i) thereby expelling interstitial fluid from said hydrogel.

In a second aspect, the invention relates to a biomaterial obtainable by a method according to the first aspect.

In another aspect, the invention relates to a biomaterial comprising a compacted nanostructured hydrogel wherein said hydrogel comprises a polysaccharide network, a fibrin network and cells embedded within said hydrogel.

In further aspects, the invention relates to a biomaterial according to the first or second aspects for use in medicine as well as for use in the treatment of a nerve damage.

## BRIEF DESCRIPTION OF THE FIGURES

**Figure 1:** Schematic representation of the method used for the nanostructuring of the biomaterial of the present invention.

**Figure 2:** Yield-stress (A), elastic modulus (B) and viscous modules (C) values of the different non-nanostructured and nanostructured bioengineered biomaterials at week 7 after the formation of the biomaterial using different fibrin and agarose concentrations.

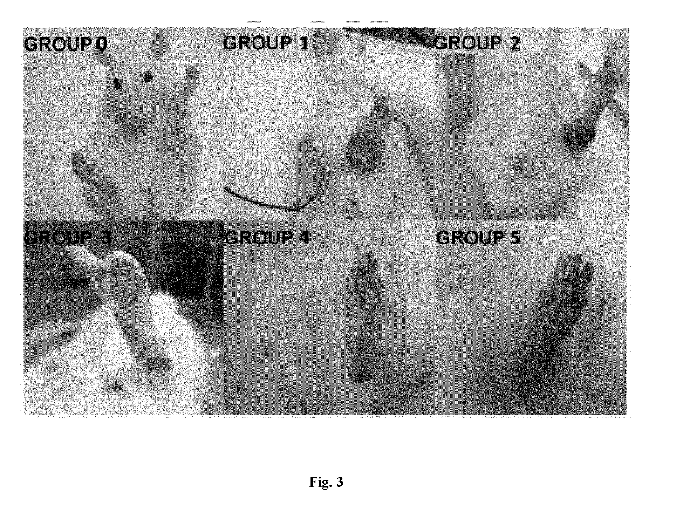

**Figure 3:** Presence of neurotrophic ulcers and self-mutilations in animals not subjected to any surgical intervention (Group 0), in animals suffering a lesion of the sciatic nerve with a one centimeter nerve defect without surgical repair thereof (Group 1), animals with a lesion of the sciatic nerve and microsurgical repair by means of a nerve guide formed by a collagen tube filled with heparinized saline (Group 2); animals with a lesion of the sciatic nerve and microsurgical repair by means of a nerve guide formed by a commercial collagen tube filled with a fibrin-agarose biomaterial (Group 3), animals with a lesion of the sciatic nerve and microsurgical repair by means of a nerve gluide formed by a commercial collagen tube filled with autologous adipose tissue-derived mesenchymal stem cells (AT-MSC) immersed in fibrin-agarose biomaterials (Group 4), and in animals with a lesion of the sciatic nerve and microsurgical repair of the nerve defect by means of implanting the artificial nerve product described in Example 1 of this specification (nanostructured artificial nerve product) (Group 5).

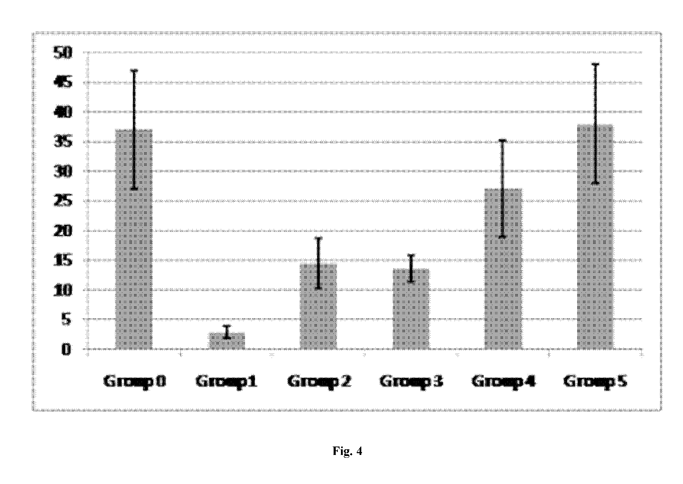

**Figure 4:** Footprint measured in millimeters for each study group (mean).

**DETAILED DESCRIPTION OF THE INVENTION**

The present invention is based on the surprising and exploitable finding that a compacted biomaterial comprising fibrin and a polysaccharide network and containing stem cells is capable of promoting regeneration of a peripheral nerve lesion more efficiently that other biomaterials known in the art. For instance, as shown in the examples of the present invention, the biomaterial of the present invention is capable of restoring both clinical parameters and electromyographic parameters in a peripheral nerve lesion more efficiently than other biomaterials. Moreover, the biomaterial is capable of restoring the neurogenic retraction (measured as the length of the footprint) in the animals which suffered a peripheral nerve lesion to values similar to those of the animals which did not suffer any lesion in the nerve.

Thus, in a first aspect, the invention relates to a method for producing a biomaterial capable of regenerating a nerve lesion comprising the steps of:

(i) providing a hydrogel comprising a polysaccharide network, a fibrin network and cells embedded within said hydrogel and

(ii) compacting the biomaterial of step (i) by expelling interstitial fluid from said hydrogel.

The term "biomaterial" as used here refers to a natural or synthetic biocompatible material that is suitable for being implanted into living tissue and which performs, augments, or replaces a natural function in a patient or subject. The biomaterial according to the present invention is suitable for regenerating a nerve lesion by regenerating the nerve tissue once it has been implanted into the site of the lesion and/or by acting as nerve guide promoting growth from the recessed ends of the severed nerve and proper orientation of said nerve ends.

The term network, as used herein, is intended to mean an association of macromolecules. This association between the macromolecules can be provided by strong or weak interactions (covalent bonds, hydrogen bonds, Van der Waals, etc.). In the case of covalent bonds, the association is a cross-linking and the network is said to be "cross-linked".

The term "polymer" is intended to mean a macromolecule, for example a protein or saccharide polymer. A polymer network is an association of peptide polymers or an association of saccharide polymers. The polymer network constitutes a gel.

A gel is composed of at least two components, one of which, which is very highly predominant, corresponds to a liquid solvent and the other of which is a component that can be described as solid. The two components are continuous throughout the entire medium. The "solid" phase constitutes a network which traps the "liquid" phase corresponding to the solvent, and prevents it from flowing. The medium as a whole behaves like a soft and elastic solid that is easy to deform.

Gels can be classified according to the type of links which form the network. Thus, two major gelling mechanisms can be distinguished, which result in "physical" gels or in "chemical" gels. Starting from a solution or from a dispersion in the liquid state, the formation of the gel is the result of the formation of a continuous solid network. This transformation is called solution/gel transition.

A physical gel is a supramolecular assembly constituted of molecules linked to one another by low-energy bonds (Van der Waals, hydrogen bonds, polar bonds, etc.). The stability of this assembly is associated with a precise range of physicochemical conditions (pH, concentration of molecules, temperature, solvent quality, ionic strength, etc.). Outside this range, the mixture is liquid. The sol/gel transition is therefore reversible for physical gels. Thus, a modification of the parameters of the medium can lead to the destruction of the structure and induce a gel/sol transition. Compositions for obtaining "physical" gels are well known from the prior art. Biogels are obtained essentially from macromolecules or polymers of natural origin: proteins or polysaccharides.

Gels described as "chemical" are also known in the prior art. A chemical gel corresponds to a supramolecular assembly, the molecules of which are associated by high-energy bonds (covalent bonds). The stability of this assembly is therefore very high. These chemical gels exhibit improved stability, the only means of performing a gel/solution transition consisting in destroying the covalent bonds of the network. For this reason, the sol/gel transition of chemical gels is said to be irreversible.

The biomaterial according to the invention comprises a fibrin network. The terms "fibrin," "fibrin matrix," "fibrin biomatrix, "fibrin-based scaffold," "fibrin scaffold," "fibrin gel," "fibrin adhesive," and "fibrin sealant" are often used interchangeably herein and in the art to refer to a three-dimensional network resulting from the polymerization product of fibrinogen in the presence of thrombin and a Ca++ source catalyzed by the enzyme Factor XIIIa. Such fibrin matrix or fibrin clot is provided naturally by the body after injury, but also can be engineered as a tissue substitute.

In order to obtain a fibrin network, fibrinogen is induced to polymerize by thrombin. The concentration of fibrinogen used to prepare the fibrin may vary and includes concentrations including, but not limited to, about 1 mg/ml, about 2 mg/ml, about 3 mg/ml, about 4 mg/ml, about 5 mg/ml, about 6 mg/ml, about 7 mg/ml, about 8 mg/ml, about 9 mg/ml, about 10 mg/ml, about 15 mg/ml, about 20 mg/ml, about 25 mg/ml, about 30 mg/ml, about 35 mg/ml, about 40 mg/ml, about 45 mg/ml, about 50 mg/ml, about 60 mg/ml, about 70 mg/ml, about 80 mg/ml, about 90 mg/ml, about 100 mg/ml, about 150 mg/ml, and up to about 200 mg/ml (final concentrations in the gels), or in intermediate concentrations as necessary. In certain aspects, the fibrinogen is added at concentrations of about 10 mg/ml and about 20 mg/ml. In a preferred embodiment, the fibrinogen is added at concentrations of about 1 to 5 mg/ml.

Further, the fibrinogen may be combined with any appropriate concentration of thrombin. Thrombin is added in varying concentrations including, but not limited to, about 0.1 IU/ml, about 0.2 IU/ml, about 0.3 IU/ml, about 0.4 IU/ml, about 0.5 IU/ml, about 0.6 IU/ml, about 0.7 IU/ml, about 0.8 IU/ml, about 0.9 IU/ml, about 1 IU/ml, about 1.1 IU/ml, about 1.2 IU/ml, about 1.3 IU/ml, about 1.4 IU/ml, about 1.5 IU/ml, about 2 IU/ml, about 3 IU/ml, about 4 IU/ml, about 5 IU/ml, about 6 IU/ml, about 7 IU/ml, about 8 IU/ml, about 9 IU/ml, about 10 IU/ml, about 11 IU/ml, about 12 IU/ml, about 13 IU/ml, about 14 IU/ml, about 15 IU/ml, about 16 IU/ml, about 17 IU/ml, about 18 IU/ml, about 19 IU/ml, about 20 IU/ml, about 21 IU/ml, about 22 IU/ml, about 23 IU/ml, about 24 IU/ml, about 25 IU/ml, about 30 IU/ml, about 35 IU/ml, about 40 IU/ml, about 45 IU/ml, about 50 IU/ml, about 60 IU/ml, about 70 IU/ml, about 80 IU/ml, about 90 IU/ml, about 100 IU/ml, about 1 10 IU/ml, about 120 IU/ml, about 130 IU/ml, about 140 IU/ml, about 150 IU/ml, about 160 IU/ml, about 170 IU/ml, about 180 IU/ml, about 190 IU/ml, about 200 IU/ml, about 225 IU/ml, about 250 IU/ml, about 275 IU/ml, about 300 IU/ml, or in intermediate concentrations as necessary.

In some aspects, the compositions of the invention comprise fibrin clot formulations (thrombin to fibrinogen ratios) in ratios ranging from about 0.001 to about 100.0. In another aspect, thrombin to fibrinogen ratios range from about 0.01 to about 10.0. In various aspects, the ratio is about 0.04, or about 0.05, or about 0.1

In particular aspects of the invention, the hydrogel comprises fibrinogen at a final concentration from about 1 mg/ml to about 100 mg/ml and thrombin at a final concentration from about 0.5 IU/ml to about 250 IU/ml. In more particular aspects of the invention, fibrin clot formulations (final concentrations of fibrinogen (mg/ml) to thrombin (international units (IU) or units (U)/ml) are about 10 mg/2U and about 20mg/4U.

Fibrinogen is a high molecular weight protein present in blood plasma. Thus, the starting material for the generation of the fibrin network may be plasma or a plasma derivatives, as such but not limited cryoprecipitate or fibrinogen concentrate. In this case, since the plasma contains all molecules needed for polymerization of fibrinogen, the fibrin network can be formed from plasma by the addition of a coagulation factor or of a source of calcium.

The term "coagulation factor" as used herein, refers to a component, usually a protein present in blood plasma and involved in the chain reaction that makes it possible clotting. Suitable clotting factors for use in the present invention include, without limitation, factor III (tissue factor or thromboplastin); factor IV; factor V (proaccelerin or labile factor); factor VI, factor VII (stable factor or proconvertin); factor VIII: (antihemophilic factor A or von Willebrand factor); factor IX (Antihemophilic B or Christmas factor), factor X (Stuart-Prower factor), factor XI (plasma thromboplastin antecedent): factor XII (Hageman factor), factor XIII (fibrin stabilizing factor), von Willebrand factor, high-molecular-weight kininogen (HMWK or Fitzgerald factor) and the like.

In a more preferred embodiment of this first aspect of the invention, coagulation of the plasma and formation of the fibrinogen matrix is carried out by the addition to the plasma of a calcium source. In a preferred embodiment, the calcium source is a calcium salt such as, without limitation, calcium chloride, calcium gluconate or a combination of both. The concentration of calcium salt should be sufficient to induce polymerization of fibrinogen. In a more preferred embodiment, the salt calcium is calcium chloride. In a more preferred embodiment, the concentration of calcium chloride in the resulting product biomaterial is between 0.25 and 3 g/L, optionally between 0.5 and 4 g / L. However, lower or higher concentrations could also be used.

In a preferred embodiment, the concentration of fibrinogen in the hydrogel as defined in step (i) is between 0.5 and 10 g/L, optionally between 1 and 10 g/L. In a more preferred embodiment, the concentration in the hydrogel is between 1 and 4 g/L, optionally between 2 and 4 g/L. However, a lower or higher concentration could also be used.

The fibrin polymer can be degraded through a process called fibrinolysis. During fibrinolysis, plasminogen is converted into the active enzyme plasmin by tissue plasminogen activator (tPA). Plasmin binds to fibrin surface through its binding sites resulting in the degradation of fibrin. To prevent fibrinolysis of the hydrogel, the hydrogel as defined in step (b) may contain and antifibrinolytic agent such as, without limitation, epsilon aminocaproic acid, tranexamic acid or aprotinin.

Tranexamic acid is a synthetic derivative of the amino acid lysine with high affinity for lysine binding sites in plasminogen, being capable of blocking these sites and preventing binding of plasminogen activator to the surface of fibrin, exerting a antifibrinolitic effect. Tranexamic acid is advantageous over other antifibrinolytic agents of animal origin in that it does not transmit diseases. Therefore, in a preferred embodiment, the agent antifibrinolytic is tranexamic acid. In a more preferred embodiment, the concentration of tranexamic acid in hydrogel is between 0.5 and 2 g / L, preferably 1 to 2 g / L. However, lower or higher concentrations could also be used.

Fibrin matrices are very versatile, so they have been used for the development of different artificial tissues. However, the clinical use of these has been limited due to the fact mainly of low consistency, its difficult handling and its great fragility. For this reason, the hydrogel used in step (a) of the method of the invention further comprises a polysaccharide network. In general, the polysaccharide is used to provide strength and consistency of the tissue, and should be soluble in it.

The term "polysaccharide network", as used herein, refers to a three-dimensional network resulting from the physical gelification of a polysaccharide. As explained above, polysaccharide networks usually form physical gels, i.e. gels which are not stabilized by chemical bonds between the components but rather by low-energy bonds (Van der Waals, hydrogen bonds, polar bonds, ionic bonds, etc.).

The polysaccharide may be any polysaccharide capable of forming a gel, preferably by a change of temperature, and may be selected from the group consisting of agarose, agar, cellulose, dextran, starch, chitosan, konjac, curdlan, carrageenan, pectin, gellan, and alginate. As the skilled person will understand, such gelled polysaccharide particles are advantageously comprised of one polysaccharide, but the present invention also embraces using particles comprised of a mixture of two or more polysaccharides. In an advantageous embodiment of the present method, the polysaccharide is agarose.

Those skilled in the art will appreciate that suitable conditions for the gelling of the polysaccharide and formation of the polysaccharide network will depend on the nature of it. Thus, in the preferred case of the use of agarose as a polysaccharide, it is sufficient to reduce the temperature below the gelling temperature of agarose concentration. This temperature can be readily determined by one skilled in the art from tables that correlate the gelling temperature of agarose concentration in the sample (for example, the table available at http://www.lonzabio.com/uploads/tx_mwaxmarketingmaterial/Lonza_BenchGuides_So urceBook_Appendix_B_-_Agarose_Physical_Chemistry.pdf

In other embodiments, the invention contemplates the use of modified agaroses including, without limitation, methylagarose, hydroxyethylagarose, hydroxypropylagarose, allylagarose, acetylagarose and the like.

In a preferred embodiment the agarose is a low melting point agarose. Low melting point agarose is commercially available such as Ultra Pure (R) agarose (Invitrogen), NuSieve (R) GTG (R) Agarose (Lopza), LM Agarose and LM Sieve Agarose (Pronadisa) Agarose SERVA Premium (Serva) and the like. In the event that the polysaccharide network is formed by agarose, the formation of the polysaccharide network is carried out at a temperature of the mixture to 10-30 °C, preferably 15-25 °C, most preferably 20-25 °C.

In a preferred embodiment, the polysaccharide network is formed by agarose. In another preferred embodiment, the concentration of agarose in the biomaterial before the compacting step is of about 0.025 to 0.3% and, preferably, 0.1%.

The hydrogel provided in step (i) of the method of the invention is therefore obtained by performing a cross-linking reaction of fibrinogen into fibrin and by inducing the gelling of the polysaccharide. The skilled person will understand that both reactions can be carried out in any order, i.e. the polymerization of the fibrinogen into fibrin can be carried out first and be followed by the gelling of the polysaccharide or the gelling of the polysaccharide can be carried out first and be followed by the polymerization of the fibrinogen into the fibrin network. However, in a preferred embodiment, the fibrin network is firstly formed as explained above (by placing a fibrinogen-comprising composition under conditions adequate for the formation of a fibrin network) and then the polysaccharide matrix is formed by contacting the first network with a solution containing the polysaccharide and placing the mixture under conditions adequate for the formation of the polysaccharide network. In the particular case that the polysaccharide network is formed by agarose, formation of the agarose hydrogel can be achieved by placing the mixture at a temperature below the melting point of the agarose. Such temperature can be determined by the skilled person without further ado by consulting the melting profile of the particular agarose type being used.

The hydrogel used in step (i) of the method for producing a biomaterial according to the invention further comprises stem cells embedded within said hydrogel.

As further component, the nanostructured biomaterial comprises cells embedded within the hydrogel. The term "cells" refers both to differentiated cells as well as to stem cells.

As used herein, "differentiated cell," means a cell with at least one phenotypic characteristic (e.g., a distinctive cell morphology or the expression of a marker nucleic acid or protein) that distinguishes it from undifferentiated cells or from cells found in other tissue types. Suitable differentiated cells that can be embedded in the biomaterial according to the invention include, without limitation, (i) blood cells, e.g. erythrocytes, reticulocytes, leukocytes, thrombocytes, megakaryocytes, mast cells, dendritic cells; (ii) immune cells, e.g. lymphoid cells: B cells, T cells (cytotoxic T cells, natural killer T cells, regulatory T cells, T helper cells), natural killer cells; myeloid cells: granulocytes (basophil granulocytes, eosinophil granulocytes, neutrophil granulocytes/hypersegmented neutrophil) monocytes/macrophages; (iii) endocrine system cells: thyroid cells (thyroid epithelial cells, parafollicular cells), parathyroid cells (parathyroid chief cells, oxyphil cells), adrenal cells (chromaffin cells), pineal cells (pinealocyte); (iv) nervous system cells: e.g. glial cells: glioblasts (astrocyte, oligodendrocyte), microglia, magnocellular neurose-cretory cells, Stellate cells, Boettcher cells, pituitary cells (gonadotrope, corticotrope, thyrotrope, somatotrope, lactotroph cells); (v) respiratory system cells, e.g. pneumocytes (Type I pneumocytes, Type II pneumocytes), Clara cells, Goblet cells, Alveolar macrophages; (vi) circulatory system cells, e.g. myocardiocytes, pericytes; (vii) digestive system cells, e.g. stomach cells (gastric chief cells, parietal cells), Goblet cells, Paneth cells, G cells, D cells, ECL cells, I cells, K cells, S cells, enteroendocrine cells, enterochromaffin cells, APUD cells, liver cells (hepatocytes, Kupffer cells); (viii) cartilage, bone and muscle cells: bone cells: osteoblasts, osteocytes, osteoclasts, teeth cells (cementoblasts, ameloblasts), cartilage cells: chondroblasts, chondrocytes, (ix) skin and hair cells: trichocytes, keratinocytes, melanocytes (Nevus cells); (x) muscle cells: myocytes, other cells: adipocytes, fibroblasts, tendon cells; (xi) urinary system cells, e.g. podocytes, juxtaglomerular cells, intraglomerular mesangial cells, extraglomerular mesangial cells, kidney proximal tubule brush border cells, macula densa cells; and (xii) reproductive system cells, e.g., male cells (spermatozoon, Sertoli cells, Leydig cells) and female cells (ovum cells).

In a preferred embodiment, the cells are selected from the group consisting of stem cells, stromal cells, neuronal cells, glial cells and combinations thereof.

The term "stromal cell", as used herein, refers to connective tissue cells that make up the support structure of biological tissues. Stromal cells include fibroblasts, immune cells, pericytes, endothelial cells, and inflammatory cells.

The term "neuronal cell", as used herein, refers to a cell exhibiting one or more phenotypic characteristics of a neuron. Neurons, include cholinergic neurons, GABAergic neurons, glutamatergic neurons, dopaminergic neurons, and serotonergic neurons, are well characterised both morphologically and biochemically. As those skilled in the art will recognise, the neuronal cells described herein refer to all cells along the neuronal differentiation pathway through to a fully differentiated dendritic neuron, such as those with fully functional axons or neurotransmitter release.

Neuronal cells may be identified as those expressing one or more markers associated with neurons or neuronal progenitor cells, including, for example, one or more of Nestin (a class VI intermediate filament protein that is routinely used as a CNS stem cell or neural progenitor marker), Musashi-1 (an intermediate filament that is expressed in CNS cells during CNS development), doublecortin, MAP-2 (a major microtubule associated protein of brain tissue and a marker of mature neuronal cells known to interact with neurofilaments, actin and other elements of the cytoskeleton), ABCG2 (ATP-binding cassette, sub-family G, member 2, reported to be a marker for pluripotent stem cells), Neurofilament-200, SMI32, Neuronal class III tubulin, Oct4, synaptophysin, various ligands, effectors and receptors in the Notch/Delta signalling pathway including Jagged 1, Jagged 2, Delta-like 1, Notch 1 receptor, and the Notch effectors Hes1 and Hes5, Neurofilament H, and tyrosine hydroxylase.

As used herein, the term "glial cell" refers to all types of central nerve system (CNS) cells that cannot receive or transmit nerve signals. Generally, "glial cells" include astrocytes, oligodendrocytes, ependymal cells and their precursors. These glial cells perform various activities that can be regarded as performing supporting, housekeeping, and "nursing" functions within the CNS. Neuroglia have high-affinity transmitter uptake systems, voltage- dependent and transmitter-gated ion channels, and can release transmitters. GHa cell-specific markers have been identified. The markers for glia cells include but are not limited to intermediate filament typical for astrocytes (GFAP), calcium-binding protein typical for astrocytes (S1OO beta), proteoglycan-component typical for glial precursors (NG2), surface antigen typical for glial precursors (A2B5), RIP for oligodendrotytes, RC2 for radial glia, 04GALC and 01 GALC for oligodendrocytes, CNP, PLP, MBP (myelin components generated by oligodendrocytes).

The terms "stem cell", "precursor cell" and "progenitor cell," and are used interchangeably in the art and as used herein refer either to a pluripotent or lineage-uncommitted progenitor cell, which is potentially capable of an unlimited number of mitotic divisions to either renew itself or to produce progeny cells which will differentiate into the desired defined cell type. In contrast to pluripotent stem cells, lineage-committed progenitor cells are generally considered to be incapable of giving rise to numerous cell types that phenotypically differ from each other. Instead, progenitor cells give rise to one or possibly two lineage-committed cell types. Stem cells are cells that on division face two developmental options: the daughter cells can be identical to the original cell (self-renewal) or they may be the progenitors of more specialised cell types (differentiation). Stem cells are therefore capable of adopting one or other pathway (a further pathway exists in which one of each cell type can be formed). Stem cells are therefore cells which are not terminally differentiated and are able to produce cells of other types. As used in this invention, the term "stem cell" particularly refers to pluripotent stem cells, particularly mammalian (e.g. human) pluripotent stem cells.

Exemplary stem cells for use in the methods herein include, but are not limited to, embryonic stem cells (ESCs), germinal stem cells and/or adult stem cells (ASCs). ASCs include, but are not limited to, hematopoietic stems cells (HSCs) from bone marrow, and other primitive progenitor cells such as mesenchymal stem cells (MSCs) and multipotent adult progenitor cells (PAPCs). The stem cells in the stem cell compositions provided herein can be of human origin or non-human origin, including, but not limited to, mouse, rat or non-human primate origin. In one example, the stem cell compositions contain ESCs. For example, the stem cell compositions can contain embryonic stem cells selected from among ACT-14, AS034, AS034.1, AS034.2, AS038, AS079, AS094, BG01, BG02, BG03, BG04, CH01, CH02, CLS1, CLS2, CLS3, CLS4, ES01, ES02, ES03, ES04, ES05, ES06, ESM01, ESM02, ESM03, FC018, FES 21, FES 22, FES 29, FES 30, GE01, GE07, GE09, GE13, GE14, GE91, GE92, hES-NCL1, HS181, HS207, HUES 1, HUES10, HUES 11, HUES 12, HUES 13, HUES 14, HUES 15, HUES 16, HUES17, HUES2, HUES3, HUES4, HUES5, HUES6, HUES7, HUES8, HUES9, KhES-1, KhES-2, KhES-3, MB01, MB02, MB03, Miz-hES1, Miz-hES10, Miz-hES11, Miz-hES12, Miz-hES13, Miz-hES14, Miz-hES15, Miz-hES2, Miz-hES3, Miz-hES4, Miz-hES5, Miz-hES6, Miz-hES7, Miz-hES8, NC01, NC02, NC03, ReliCellhES1, RH1, RH3, RH4, RH5, RH6, RH7, RL05, RL07, RL10, RL13, RL15, RL20, RL21, Royan H1, SA001, SA002, SA002.5, SA046, SA085, SA111, SA121, SA142, SA167, SA181, SA191, SA196, SA202, SA203, SA211, SA218, SA240, SA279, SA348, SA352, SA399, SA611, SI-100, SI-101, SI-102, SI-103, SI-104, SI-105, SI-106, SI-107, SI-108, SI-109, SI-110, SI-111, SI-114, SI-115, SI-122, SI-123, SI-124, SI-125, SI-126, SI-128, SI-130, SI-131, SI-132, SI-133, SI-134, SI-135, SI-137, SI-138, SI-139, SI-140, SI-141, SI-144, SI-145, SI-146, SI-148, SI-149, SI-15, SI-150, SI-151, SI-153, SI-154, SI-155, SI-156, SI-157, SI-158, SI-159, SI-160, SI-161, SI-162, SI-163, SI-164, SI-165, SI-167, SI-168, SI-169, SI-170, SI-171, SI-172, SI-174, SI-175, SI-176, SI-177, SI-178, SI-179, SI-18, SI-180, SI-182, SI-183, SI-184, SI-185, SI-186, SI-187, SI-188, SI-189, SI-191, SI-192, SI-193, SI-194, SI-195, SI-196, SI-197, SI-198, SI-199, SI-200, SI-201, SI-202, SI-203, SI-204, SI-205, SI-206, SI-208, SI-209, SI-21, SI-210, SI-211, SI-213, SI-214, SI-215, SI-216, SI-217, SI-221, SI-24, SI-27, SI-28, SI-31, SI-33, SI-53, SI-60, SI-62, SI-63, SI-79, SI-80, SI-81, SI-93, SI-94, SI-95, SI-96, SI-97, SI-98, SI-99, SNUhES1, SNUhES2, SNUhES3, TE-03, TE-04, TE-06, TE-07, TE-32, TE-33, TE-62, TE-72, UC01, UC06, VAL-1, VAL-2, VAL-3, VAL-4, WA01, WA07, WA09, WA13 or WA14 cells.

The term "mesenchymal stem cells" or "MSCs" used herein refers to multipotent stem cells derived from adult tissues including but not limited to bone marrow, adipose tissue, muscle tissue, dental pulp of deciduous baby teeth, umbilical cord blood, or Wharton's jelly and which are capable of differentiating into more than one specific type of mesenchymal or connective tissue (i.e., tissues of the body which support specialized elements; e.g., adipose, osseous, stroma, cartilaginous, elastic and fibrous connective tissues).

In a preferred embodiment, the mesenchymal stem cells are adipose tissue stem cells (or ASCs). The ASCs may be derived from adipose tissue of any suitable origin, but is most preferably of human origin. It is preferred that said cells are obtained from non-pathological mammalian sources, preferably post-natal (e. g., rodent; primate). However, particularly preferred are subcutaneous adipose tissue or organ associated adipose tissue (for example but not limited to adipose associated with the heart, liver, kidneys or pancreas).

The immunophenotype of ASCs changes progressively, depending on culturing procedures (i.e. passage number). The adherence to plastic and subsequent expansion of human ASCs selects for a relatively homogeneous cell population, enriching for cells expressing a "stromal" immunophenotype, as compared to the heterogeneity of the crude stromal vascular fraction. ASCs also express stem-cell associated markers including, but not limited to, human multidrug transporter (ABCG2) and aldehyde dehydrogenase (ALDH).

The immunophenotype of ASCs can be exploited to serve as unique identifiers for ASCs. That is, the unique cell surface markers on the cells of interest can be used to isolate a specific sub-population of cells from a mixed population of cells derived from adipose tissue. Cell surface markers for BMSCs have been characterized and include expression of the following cell surface markers: CD9, CD10, CD13, CD29, CD44, CD49d, CD54, CD55, CD59, CD71, CD73, CD90, CD105, CD106, CD146, CD166, alpha -smooth muscle actin, collagen type 1, collagen type III, HLA-ABC, nestin, osteopontin, osteonectin and vimentin.

Human adipose tissue is a rich source of stromal-like adult stem cells. The cells can be isolated from lipoaspirates using known methods such as, without limitation, the methods described by Hauner and others (Hauner et al., 1989, J. Clin. Invest. 84(5):1663-1670; Hauner et al., 1988, Horm. Metab. Res. Suppl. 19:35-39), reproducible and efficient methods have been developed to isolate adult stem cells from human liposuction tissue (Aust et al., 2004, Cytotherapy 6(1):7-14; Halvorsen et al., 2001, Metabolism 50: 407-413; Mitchell et al., Stem Cells online Jan. 12, 2006:2005-0235).

The term "embedded", as used herein, refers to cells found within the three dimensional structure of the biomaterial so that they are distributed interstially within the hydrogel. Embedded cells differ from cells "seeded" on a preformed gel wherein the cells are mostly or exclusively present on the surface of the biomaterial. Embedded cells can be distinguished from seeded cells by the inability of a labeled reagent showing affinity towards the cells and having a size larger that the pores of the biomaterial to bind to the cells, whereas the same reagent is capable to detect seeded cells found on the surface of the biomaterial. Thus, in preferred embodiment, at least 100%, 99%, 98%, 97%, 96%, 95%, 94%, 93%, 92%, 91%, 90%, 80%, 70%, 60% or 50% are not accessible to a specific affinity reagent.

The present invention also contemplates that the cells which are embedded in the biomaterial are mixed populations of one or more of the above types of the cells such as:

- stromal cells and stem cells
- stromal and neuronal cells
- stromal and glial cells
- glial and neuronal cells
- stromal, glial and neuronal cells.
- stromal, glial, neuronal and stem cells
- stromal, glian, neuronal and stem cells

The mixed cells populations may comprise different ratios of the different cells. In particular, when the cell population comprise two different types of cells, then the cells may be present in a 1:9 ratio, a 2:8 ratio, a 3:7 ratio, a 4:6 ratio, a 5:5 ratio, a 6:4 ratio, a 7:3 ratio, a 8:2 ratio or 9:1 ratio.

The cells may be embedded in any arrangement. For example, the cells may be distributed homogeneously throughout the gel or distributed in defined zones, regions or layers within the gel. The cells are preferably seeded into the biomaterial when the gel is cast. Cells may be seeded within the biomaterial by mixing them with the fibrinogen-comprising composition prior to the formation of the fibrin network or within the polysaccharide solution prior to the formation of the polysaccharide gel and then allowing the biomaterial to form. Seeding of the cells is preferably performed under suitable conditions of temperature, pH, ionic strength and sheer to maintain cell viability.

The initial cell density in the gel may be from about $1 \times 10^4$ to $1 \times 10^7$ cells per ml, more preferably from about $5 \times 10^5$ to $1 \times 10^6$ cells per ml. In those cases wherein the cell suspension does not have sufficient cell density to produce a biomaterial with the desired cell density, the method according to the invention may further comprise an additional step prior to the compaction step wherein the biomaterial is maintained under conditions adequate for proliferation of the stem cells. In this case, the material may be seeded with a smaller number of cells (preferably between 10.000 - 15.000 cells/ml).

Suitable conditions for proliferation of the stem cells include, covering the biomaterial with culture medium for about 1 to about 14 days and, most preferably, 7 days at a temperature of 37°C in the presence of a suitable growth factor, a cytokine, a combination of growth factors, a combination of cytokines or a combination of growth factors and cytokines. Non-limiting examples of growth factors include stem cell factor (SCF), thrombopoietin (TPO), erythropoietin (EPO), granulocyte-macrophage colony stimulating factor (GM-CSF), granulocyte colony stimulating factor (G-CSF), macrophage colony stimulating factor (M-CSF), FLT-3/FLK-2 ligand, stromal cell-derived factor- 1 (SDF-I), tumor necrosis factor-alpha (TNF-a), transforming growth factor beta (TGF beta), interleukin 1, interleukin 2, interleukin 3, interleukin 4, interleukin 5, interleukin 6, interleukin 7, interleukin 8, interleukin 11, interleukin 12, interleukin 13, and combinations thereof. Culturing of stem cells under proliferating conditions is described in U.S. Pat. Nos. 6,511,958, 6,436,704, 6,280,718, 6,258,597, 6,184,035, 6,132708 and 5,837,5739.

Proliferation of the cells can be determined by the skilled person using routine experimentation such as by using fluorescent dyes (e.g. carboxyl-fluorescein-succinimidyl ester (CFSE)5 carboxyfluorescein succinimidyl ester diacetate (CFDA SE), carboxylic acid diacetate succinimidyl ester (DFFDA, SE), calcein, PKH, Dapi, Hoechst, thiazole orange, or propidium iodide), BrdU, radio-labelled analogues (e.g. 3H-thymidine), and agents capable of detecting cellular markers of proliferation (e.g. Ki-67, microchromosome maintenance protein 2 (Mcm2/BM28), minichromosome maintenance protein 6 (Mcm6)) such as antibodies.

The proliferation of the cells is maintained until a sufficient density of cells is reached in the hydrogel. Suitable cell densities include, without limitation, from about 20 to about 100 cells/mm$^2$, preferably from about 30 to 90 cells/mm$^2$, preferably from about 40 to 80 cells/mm$^2$, preferably from about 50 to 70 cells/mm$^2$.

In yet another embodiment, wherein the fibrin network is prepared from blood plasma, the plasma and the cells (preferably the mesenchymal stem cells) are autologous (from the same subject) or allogeneic (from a genetically different subject from the same species).

In a second step, the method for the preparation of a biomaterial according to the invention comprises compacting the biomaterial of step (i) thereby expelling interstitial fluid from said hydrogel.

Without wishing to be bound by any theory, the compacting step is thought to induce a series of three-dimensional irreversible interfibrillar reactions able to modify the biomechanical properties of the biomaterial. This process will be referred to hereinafter as "nanostructuration".

The term "compacting", as used herein, means deforming an object, such as a gel, until its volume has been reduced and in a way that the object substantially retains its new volume, even after the cause of compaction is removed. Compaction is a rapid, cell independent process which results from subjecting the gel to a physical treatment, such as an external force or pressure, which expels interstitial liquid from the gel. Compaction is distinct from the slow process of cell-driven contraction, which occurs through the intrinsic action of cells growing within the gel i.e. compaction is not cell-mediated and does not occur through the action of cells which are cultured within the gel. Cell-mediated contraction is only capable of producing a small fraction of the compaction caused by compaction as described herein. Furthermore, cellular contraction occurs with random vectors so the overall contracting vector is an average for the whole cell population. Compaction employs a vector in one, two or more defined directions and the direction, rate and extent of the compaction is controllable.

Compaction allows the cell content, matrix density and fluid content of the biomaterial to be precisely defined. Furthermore, local enhancement of one or more of these parameters within the biomaterial is possible and the spatial position and pattern of these local enhancements is controllable, for example to produce layering, zoning or channelling.

This process of compacting results in artificial tissues that show unexpectedly advantageous properties with respect to non-compacted biomaterials. In particular, biomaterials subjected to compacting process according to the present invention exhibit (i) a significant improvement in the biomechanical properties of the tissue, which allows the manipulation of the compacted tissue and represents a substantial and unexpected improvement of the rheological properties of the biomaterial, and, in particular, an increased resistance and higher elasticity, (ii) a substantial improvement of the manipulability of compacted tissue, facilitating the surgical manipulation, thus facilitating the suture to the recipient bed and implantation in experimental animals, and (iii) improving clinical outcome when implanted in laboratory animals related one hand, with the better and superior clinical efficacy of the implant due to the enhanced biomechanical properties of the biomaterial and on the other hand, the fact that compaction of biomaterials yields a higher density of fibers per

mm$^2$, and therefore have slower degradation rate by the host organism.

Unconfined compaction of a gel expels the interstitial liquid and it does not return on removal of the load: i.e. the gel undergoes a compaction. The scaffold matrix in the untreated gel is generally in a gross, hydrated form. Compaction of the gel collapses the scaffold structure without loss of structural detail, dehydrating the scaffold in the gel, and leading to increased density and strength. The interstitial liquid is typically an aqueous liquid. For example, the liquid may be water with solutes such as salts and proteins dissolved therein. In some embodiments, the interstitial liquid is a cell culture medium suitable for the growth and proliferation of cells.

In a preferred embodiment, the compacting step involves the dehydration and/or mechanical compression of the hydrogel defined in step (i). The objective of step (ii) is to produce a structural change between the fibrin fibers and the polysaccharide molecules in the biomaterial in order to achieve optimal levels of consistency and elasticity, which are not obtained by other methods described in the state of the art. The end result is an irreversible modification of the fibers which creates very favorable biomechanical qualities for surgical manipulation and for their use as clinical implants.

The term "dehydration" refers to a partial and/or complete removal of the interstitial fluid of the hydrogel. For example, the amount of interstitial fluid removed from the hydrogel may be of at least 10%, at least 20%, at least 30%, at least 40%, at least 50%, at least 60%, at least 70%, at least 80% to least 90% or at least 99% of the interstitial fluid originally contained in the hydrogel.

The removal of the interstitial fluid will result in an increase in the concentrations of the different components. Thus, if the amount of interstitial fluid removed from the hydrogel is of 20%, the concentration of the different components in the resulting hyrogel will also show an increase of 25%. If the amount of interstitial fluid removed from the hydrogel is of 30%, the concentration of the different components in the resulting hydrogel will also show an increase of about 42%. Thus, if the amount of interstitial fluid removed from the hydrogel is of 40%, the concentration of the different components in the resulting hydrogel will show an increase of about 66%.

In a preferred embodiment, the combined compaction-dehydration step is carried out until the hydrogel has lost at least about 40% of the initial interstitial fluid content, preferably about 40% of the interstitial fluid. This results in an increase in the concentration of the different components of about 1.66 times (or 66%) with respect to the material prior to the compaction step. Thus, if the agarose concentration in the hydrogel prior to the compaction/dehydration step is of 0.025% to about 0,3%, the concentration after the compaction/dehydration step will be of about 0,04% to about 0,5%.

In a preferred embodiment, the biomaterial contains 0.1% of the polysaccharide, preferably agarose, prior to the compaction step and thus, if the compaction results in the loss of approximately 40% of the interstitial fluid, the concentration of polysaccharide, preferably agarose, in the compacted biomaterial will be of about of 0.16%.

The cell density in the biomaterial after the compaction step is about 80 to 100 cells/mm$^2$.

The reduction in gel volume caused by compaction will be inversely related to the increase in cell density within the gel. Thus, compaction of the gel as described herein may increase the cell density by 5 to 200 fold, more preferably 10 to 100 fold.

The amount or extent of compaction may be varied, depending on the application of the biomaterial. Compaction of the gel, for example by compression, may result in a reduction in one or more dimensions of the gel of at least 5 fold, at least 10 fold or at least 20 fold. The one or more dimensions may be reduced by 200 fold or less, 150 fold or less, or 100 fold or less. In preferred embodiments, the thickness of the gel is reduced by compaction. For example, the volume of the gel may be reduced by 50% or more, 60% or more, 70% or more, 80% or more, 90% or more, 95% or more, 99% or more, or 99.9% or more by compaction.

Stiffness, or modulus, is a physical material property to be considered for applications in regenerative medicine and tissue engineering. In most cases, it is preferable to have the material's modulus close to that of the target tissue to avoid possible stress-shielding effects and maintain sufficient mechanical support during in vitro and/or in vivo cell growth and tissue-remodeling processes. In a preferred embodiment, the compacting step is carried out until the compacted hydrogel shows a yield-stress value of about 5.8 Pa to 38.8 Pa. In yet another embodiment, the compacting step is carried out until the yield-stress of the biomaterial increases at least 6 times, preferably between 6 and 10 times.

In another embodiment, the compacting step is carried out until the compacted hydrogel shows a elastic modulus of 22 to 355 Pa. In yet another embodiment, the compacting step is carried out until the elastic modulus of the biomaterial increases at least 2 times, preferably between 2 and 5 times.

In another embodiment, the compacting step is carried out until the compacted hydrogel shows a viscous modulus of 4 to 27 Pa. In yet another embodiment, the compacting step is carried out until the viscous modulus of the biomaterial increases at least 4 times, preferably between 4 and 5 times.

The time required for compaction will be less than the time required for cell-driven contraction to occur and will vary in accordance with the compaction method and the conditions used. For example, compaction may occur in less than 12 hours, less than 6 hours, less than 3 hours, less than 1 hour, less than 30 minutes of less than 10 minutes. In some preferred embodiments, the gel may be compacted in 2 minutes or less, or 1 minute or less.

Compaction of the gel may be associated with the loss or removal of some or all of the interstitial fluid from said gel. For example, the amount of fluid lost or removed from the gel by compaction may be at least 50%, at least 60%, at least 70%, at least 80%, at least 90%, at least 99%, or at least 99.9% of the 10 original fluid content of the gel.

Preferably, some interstitial fluid remains after compaction, for example at least 10%, at least 1% or at least 0.1% of the original fluid content of the gel. In preferred embodiments, the gel is not subjected to drying or desiccation, for example heat-, freeze-, airflow or vacuum drying, following compaction, as dehydration kills cells and damages biomaterial structure.

The cell density after compaction may be from $1 \times 10^7$ to $1 \times 10^9$ cells per ml, more preferably from $5 \times 10^7$ to $5 \times 10^8$ cells per ml.

The proportion of cells in the compacted biomaterial will depend partly on cell size. Small cells, such as red blood cells, may represent a lower proportion of the compacted biomaterial relative to larger cells, such as fibroblasts, even when present at the same cell densities. In general, cells may represent at least 1% v/v, at least 5% v/v or at least 10% v/v of the compacted biomaterial. For optimal physical properties, a biomaterial construct may comprise less than 50% v/v, less than 40% v/v or less than 30% v/v cells.

Cells in the compacted biomaterial are susceptible to desiccation. To reduce cell death and/or damage associated with desiccation, the gel may be compacted in an aqueous liquid, for example a culture medium, such as DMEM, Ham's or Eagle's medium or a physiological buffer such as Ringer's or PBS. For non-cellular biomaterials, any solvent compatible with the scaffold matrix may be used.

Cells are also susceptible to hypoxic cell death and/or damage due to the high cell densities within the compacted biomaterial. To reduce and/or prevent cell death or damage, an implant or biomaterial may be stored under conditions which maintain viability but which do not support cell growth, until ready for use. For example, the implant or biomaterial may be stored at low temperature, such as 0 to 5°C, preferably 4°C.

Dehydration of the hydrogel can be achieved by any physical or chemical procedure. In a preferred embodiment, the dehydration is carried out by a selected from the list including drainage, evaporation, suction, capillary pressure, osmosis and electro-osmosis.

The interstitial fluid can be removed by tilting the hydrogel, whereby the interstitial fluid drains due to gravity and the gradient.

The liquid may also be removed by suction, for example, by applying a vacuum using a mechanical pump to the surface of the hydrogel. The interstitial fluid can also be removed by evaporation, for example, by incubating the hydrogel under conditions that promote evaporation, for example, at a pressure lower than atmospheric pressure and/or a temperature higher than room temperature.

The interstitial fluid can also be removed using an osmotic agent with a tendency to absorb water, such as, without limitation, a hyperosmolar sodium chloride solution., The hydrogel can be separated from the hyperosmotic solution through a semipermeable membrane, a blotting sponge or other material.

In a preferred embodiment, the interstitial fluid can be removed by capillary pressure, for example, by applying an absorbent material to the hydrogel. Examples of absorbent material that could be used in step (ii) of the invention for effecting the dehydration include, without limitation, filter paper, 3M Whatman paper, cellulose fiber or absorbent cloth. Preferably, the absorbent material is sterilized.

The time required for drying will depend on the procedure or procedures used, and can be determined easily by an expert in the field. The suitability of artificial tissue obtained through the use of a particular procedure of dehydration during a certain period of time can be determined by various methods of evaluation known in the prior art such as, without limitation, those described in the examples in this report.

Interstitial fluid may also be removed by mechanical compression of the hydrogel. Mechanical compression of the hydrogel can also serve to give the resulting product of step (ii) a defined shape desired.

Compression of the hydrogel can be performed by any procedure known in the state of the art. Thus, compression may be a "static" compression, where the hydrogel remains stationary, for example, but not limited to, applying a static load (for example, a dead weight), a hydraulic or cam. The compression may also be a "dynamic" compression where the hydrogel moves during compression, for example, by applying one or more rollers or by extrusion through a constricting orifice.

Mechanical compression of the hydrogel can be made by extrusion, for example, by passing the hydrogel through a hole that constrains the hydrogel, e.g. a conical chamber. The conical chamber may have porous walls, so that the removal of interstitial fluid of the hydrogel as it passes through is facilitated.

The compression of the hydrogel can be accomplished by centrifugation of the hydrogel. For example, the hydrogel can be placed on a tube with a porous bottom, so that, upon centrifugation, in addition to mechanical compression, the interstitial fluid will be expelled from the hydrogel.

In a preferred embodiment, the compression of the hydrogel is carried out by applying a weight on top of the hydrogel, so that it exerts a mechanical pressure on the tissue. It is evident that the greater the weight the less time needed to obtain an artificial tissue with the appropriate characteristics. The weight used for compression can have a flat surface or can be placed on a material having a flat surface, for example, plastic, ceramic, metal or wood.

In a preferred embodiment, the compaction step is carried out by applying a weight in top of the hydrogel. The weight that can be applied ranges from 1000 to 5000 N/m², from 2000-4000 N/m², from 2500-3500 N/m² for about 1-10 min, more preferably 2-9 min, more preferably 3-8 min, more preferably 4-7 min, more preferably 3-6 min and more preferably 3-5 min. In a still more preferred embodiment, the weight is applied for 3-5 min. In a still more preferred embodiment, the compaction step is carried out with a weight of 2000 N/m² for about 3-5 min.

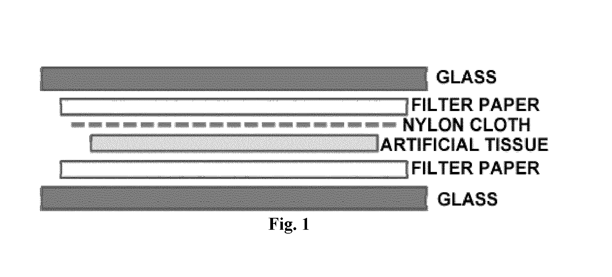

Figure 1 of this invention is an schematic drawing of a possible way of compacting the hydrogel. According to this exemplary embodiment, the hydrogel (artificial tissue) is placed between two sterile filter papers and placing over the cassette a weight of approximately 250 g (equivalent to approximately 2,000 N/m²) over a flat sterile glass surface for about 10 minutes. Between the tissue and the filter paper on which it is placed, a porous material can also be provided to prevent the hydrogel from adhering to the filter paper. The material used to prevent adhesion must be porous, in order to facilitate the outflow of water from the tissue towards the filter paper. Said material used to prevent adhesion can be, without limitation, a polycarbonate membrane, nylon, glass, ceramic or a perforated metal.

In a preferred embodiment, the compression of the hydrogel comprises applying a pressure on it. Preferably, the magnitude of the pressure is between 1,000 and 5,000 N/m², more preferably between 1,500 and 2,500 N/m², and even more preferably about 2,000 N/m². The application of this pressure can be manual, automatic or semiautomatic. The time needed to exert pressure depends on the magnitude of the applied pressure and can easily be determined by an expert in the field. Clearly, the higher the pressure the shorter the time required to obtain an artificial tissue with the appropriate characteristics. The suitability of artificial tissue obtained by applying a certain amount of pressure for a certain period of time can be determined by various methods of evaluation known in the prior art such as, without limitation, those described in the examples this invention.

The compaction of the hydrogel may be carried out by one or more procedures, being said procedures applied sequentially or simultaneously. The time required for the compaction step may be less than 12 hours, less than 6 hours, less than 3 hours, less than 1 hour, less than 30 minutes, less than 10 minutes, less than 2 minutes or less than 1 minute. The time required for the compaction depends on the procedure or procedures used, and can be determined easily by an expert in the field. The suitability of artificial tissue obtained through the use of a particular procedure for a certain period of time can be determined by various methods of evaluation known in the prior art such as, without limitation, those described in the examples in this report.

Methods described herein may be conveniently performed using a mechano-bioreactor adapted to apply suitable mechanical loads (e.g. vectored compression and tension, preferably as part of a preprogrammed sequence) to a gel, for example as part of an integrated, automated culture process. The mechano-bioreactor is preferably adapted to enable culturing and/or incubation of a three-dimensional tissue equivalent implant and may, for example, comprise uniaxial tensing means (conferring for example, cyclic or ramp load tension on a construct) and optical and electrochemical (02 plus glucose) sensors. Alternatively, a tissue equivalent implant (cellular or abiotic) may be made using a continuous flow-through process (of the "conveyor belt" type) with a sequence of steps, including the compaction step.

The biomaterial according to the invention can have various sizes and shapes. For example, the biomaterial can suitably have an elongated shape, and more suitably can be substantially cylindrically shaped (e.g., substantially rod-shaped).

In a preferred embodiment, the compacted material obtained in step (ii) is further processed by rolling a sheet of the material on itself until at least one edge of the sheet of the biomaterial overlaps with the opposite edge, thus obtaining a biomaterial of tubular shape and which is referred to hereinafter as the tubes of the invention. The rolled construct or tube can then be directly implanted in the nerve lesion. Preferably, the diameter of the rolled construct is similar to the diameter of the nerves which has to replace. Thus, in particular embodiments, the diameter of the tube is about at least 0.1 mm, at least 0.2 mm, at least 0.3 mm, at least 0.4 mm, at least 0.5 mm, at least 0.6 mm, at least 0.7 mm, at least 0.8 mm, at least 0.9 mm, at least 1 mm, at least 2 mm, at least 3 mm, at least 4 mm, at least 5 mm, at least 6 mm, at least 7 mm, at least 8 mm, at least 9 mm, at least 10 mm, at least 11 mm, at least 12 mm, at least 13 mm, at least 14 mm, or at least 15 mm. In a preferred embodiment, the diameter of the rolled construct is about 2-4 mm.

Typically, when intended for use as implant for nerves, the tubes of biomaterial according to the invention have a diameter which is similar than the diameter of the nerve to be treated. Thus, the rolled sheets of the biomaterial of the invention will have a diameter which is about 100% of the diameter of the nerve wherein it is implanted. In other embodiments of the invention, the tube will have a diameter which is less than 90 %, preferably less than 80 %, more preferably less than 70 %, more preferably less than 60 %, and most preferably less than 50 % of the diameter of the nerve. In other embodiments of the invention, the tube will have a diameter which is at least 110 percent, preferably at least 120 %, more preferably at least 130%, more preferably at least 140% percent, and most preferably at least 150% percent of the diameter of the nerve.

The tubes may have various lengths depending on the site of application and on the length of the nerve lesion. Thus, the tube can range in length from about 1 mm to about 50 cm. In particular embodiments, the length of the tube is about 1 mm, at least 2 mm, at least 3 mm, at least 4 mm, at least 5 mm, at least 1 cm, at least 2 cm, at least 3 cm, at least 4 cm, at least 5 cm, at least 6 cm, at least 7 cm, at least 8 cm, at least 9 cm, at least 10 cm, at least 50 cm or more.

As used herein, the term "sheet" does not imply any particular shape, but includes flat layers or other thin shaped objects.

The rolling of the biomaterial on itself is carried out until at least both edges have some a certain degree of overlap.

In a preferred embodiment, the rolling of the sheet is carried out to obtain a tubular structure of spiral section.

The term "spiral", as used herein, is used in accordance with its well known meaning, i.e., it refers to a shape that has at least a first end and a second end, and in which travelling along the spiral shape in the most direct route from the first end to the second end would result in winding around a pole and gradually receding from the pole (e.g., an axis). A spiral shape can be a regular spiral shape or a non- regular spiral shape like a stretched spiral.

A spiral shape can be graphically represented by a line winding around a pole, wherein the pole is the intersection of a longitudinal axis of the tube and the sectional plane, which continuously overlaps following a regular or a irregular path. Such line is the sectional view of the sheet of the tube.

The rolling of the material can be carried out in order to produce a material having a spiral shape, wherein the number of rotations in the spiral is at least 1, at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 75, at least 80, at least 85, at least 90, at least 95, at least 100 or more.

In various embodiments, the edges are attached to one another using sutures, a biologically compatible adhesive, or a combination of both, to form a fluid-tight seam extending longitudinally along the length of the graft. In some embodiments, the edges of the rolled sheet are secured using heat and pressure treatment. Suitable sutures include, for example, polypropylene sutures (PROLENE™), and can be continuous or interrupted. Suitable adhesives include, for example, fibrin glue, cyanoacrylate-based tissue adhesives (e.g., DERMABOND™) and chitosan tissue adhesives. In some embodiments, the edges of the sheet are crosslinked (e.g., using chemical or radiation induced cross-linking) to each other or to an underlying layer of material to ensure that the edges do not come loose after the sheet is rolled in a tubular construct.

The rolling of the biomaterial in order to obtain the tubular structure may be carried out under conditions that the inner lumen communicate the openings at each end of the tube. As used herein, the term "lumen" refers to the channel within a tubular structure. However, in a preferred embodiment, the biomaterial is rolled up so that there is no detectable lumen in the tube.

<u>Biomaterials of the invention</u>

The method as defined above allows the production of a biomaterial which can be used as implant for the treatment of nerve injuries. Thus, in another aspect, the invention relates to a biomaterial obtained by a method as defined above.

In another aspect, the invention relates to a biomaterial comprising a nanostructured hydrogel wherein said hydrogel comprises a polysaccharide network, a fibrin network and cells embedded within said hydrogel.

The terms "biomaterial", "compacted", "polysaccharide network", fibrin network" and "cells" have been described above in detail in the context of the method for producing a biomaterial according to the invention and are used with the same meaning in the context of the biomaterial according to the invention.

In a preferred embodiment, the polysaccharide-based hydrogel comprising fibrin and stem cells is obtained by cross-linking a fibrinogen-comprising material in the presence of the polysaccharide under conditions adequate for gelification of the polysaccharide.

In another preferred embodiment, the fibrinogen-comprising material is blood plasma. In yet another preferred embodiment, the cross-linking of the fibrinogen-comprising material is carried out in the presence of a coagulation factor, a calcium source and, optionally, in the presence of an antifibrinolytic agent. In a more preferred embodiment, the coagulation factor is thrombin. In yet another embodiment, the antifibrinolytic agent is tranexamic acid. In yet another preferred embodiment, the calcium source is a calcium salt and, most preferably, calcium chloride.

In another embodiment, the polysaccharide is agarose. In a still more preferred embodiment, the agarose is low-melting point agarose. In yet another preferred embodiment, the concentration of agarose in the biomaterial is of about 0.04%-0.5%, preferably of about 0.16%

In another embodiment, the cells are selected from the group consisting of stem cells, stromal cells, glial cells and neuronal cells. In a preferred embodiment, the cells are stem cells. In yet another embodiment, the stem cells are mesenchymal stem cells. In a still more preferred embodiment, the mesenchymal stem cells are adipose tissue derived mesenchymal stem cells. In yet another embodiment, when the biomaterial has been obtained by using plasma, the stem cells and the plasma are autologous.

In a preferred embodiment, the density of cells in the biomaterial may be from about $1 \times 10^4$ to $1 \times 10^7$ cells per ml, more preferably from about $5 \times 10^5$ to $1 \times 10^6$ cells per ml. In another preferred embodiment, the cell density in the compacted hydrogel include, without limitation, from about 20 to about 100 cells/mm$^2$, preferably from about 30 to 90 cells/mm$^2$, preferably from about 40 to 80 cells/mm$^2$, preferably from about 50 to 70 cells/mm$^2$

In a preferred embodiment, the compacted hydrogel of the invention shows a yield-stress value of about 5.8 Pa to 38.8 Pa. In yet another embodiment, the compacted hydrogel shows a elastic modulus of 22 to 355 Pa. In another embodiment, the compacted hydrogel shows a viscous modulus of 4 to 27 Pa.

In a preferred embodiment, the biomaterial has a spiral section.

The biomaterial according to the invention may optionally contain one or more active ingredients such as one or more growth factors (e.g., in an amount ranging from 0.0000001 to 1 or 5 percent by weight of the matrix composition) to facilitate nerve growth. Examples of suitable active ingredients include, but are not limited to, nerve growth factor, vascular endothelial growth factor, fibronectin, fibrin, laminin, acidic and basic fibroblast growth factors, testosterone, ganglioside GM-I, catalase, insulin-like growth factor-I (IGF-I), platelet-derived growth factor (PDGF), neuronal growth factor galectin-1, and combinations thereof. See, e.g., U.S. Patent No. 6,506,727 to Hansson et al. and U.S. Patent No. 6,890.531 to Horie et al. As used herein, "growth factors" include molecules that promote the regeneration, growth and survival of nervous tissue. Growth factors that are used in some embodiments of the present invention may be those naturally found in keratin extracts, or may be in the form of an additive, added to the keratin extracts or formed keratin matrices. Examples of growth factors include, but are not limited to, nerve growth factor (NGF) and other neurotrophins, platelet-derived growth factor (PDGF), erythropoietin (EPO), thrombopoietin (TPO), myostatin (GDF-8), growth differentiation factor-9 (GDF9), basic fibroblast growth factor (bFGF or FGF2), epidermal growth factor (EGF), hepatocyte growth factor (HGF), granulocyte-colony stimulating factor (G-CSF), and granulocyte-macrophage colony stimulating factor (GM-CSF). There are many structurally and evolutionarily related proteins that make up large families of growth factors, and there are numerous growth factor families, e.g., the neurotrophins (NGF, BDNF, and NT3). The neurotrophins are a family of molecules that promote the growth and survival of nervous tissue. Examples of neurotrophins include, but are not limited to, nerve growth factor (NGF), brain-derived neurotrophic factor (BDNF), neurotrophin 3 (NT-3), and neurotrophin 4 (NT-4). See U.S. Pat. Nos. 5,843,914 to Johnson, Jr. et al.; 5,488,099 to Persson et al.; 5,438,121 to Barde et al.; 5,235,043 to Collins et al.; and 6,005,081 to Burton et al.

Moreover, the biomaterial according to the invention may optionally comprise one or more immunomodulatory compounds. As used herein an "immunosuppressive or immunomodulatory agent" is an agent that generally or specifically suppresses or modulates a mammalian immune response

As used herein, the term "immunomodulator" includes cytokines, lymphokines, monokines, stem cell growth factors, lymphotoxins, hematopoietic factors, colony stimulating factors (CSF), interferons (IFN), parathyroid hormone, thyroxine, insulin, proinsulin, relaxin, prorelaxin, follicle stimulating hormone (FSH), thyroid stimulating hormone (TSH), luteinizing hormone (LH), hepatic growth factor, prostaglandin, fibroblast growth factor, prolactin, placental lactogen, OB protein, transforming growth factor (TGF), TGF-$\alpha$, TGF-$\beta$, insulin-like growth factor (IGF), erythropoietin, thrombopoietin, tumor necrosis factor (TNF), TNF-$\alpha$, TNF- beta, mullerian-inhibiting substance, mouse gonadotropin- associated peptide, inhibin, activin, vascular endothelial growth factor, integrin, interleukin (IL), granulocyte-colony stimulating factor (G-CSF), granulocyte macrophage-colony stimulating factor (GM-CSF), interferon-a, interferon- beta, interferon-$\gamma$, SI factor, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18 IL-21, IL-25, LIF, kit-ligand, FLT-3, angiostatin, thrombospondin, endostatin, LT, and the like.

The biomaterial according to the invention may also contain an immune modulator cells, in particular mesenchymal stem cells and, more preferably, adipose stem cells.

Therapeutic methods of the invention

In another aspect, the invention relates to a biomaterial of the invention for use in medicine.

In another aspect, the invention relates to a biomaterial according to the invention for use in the treatment of a nerve damage. In another aspect, the invention relates to a method for the treatment of nerve damage in a subject in need thereof which comprises the administration to said subject of a biomaterial according to the invention. Additionally, the invention relates to the use of a biomaterial according to the invention for the manufacture of a medicament for the treatment of nerve damage.

When used for the treatment of a nerve lesion, the biomaterial can act as a graft. As used herein, a "graft" refers to a cell, tissue, organ, scaffold, and the like that is implanted into an individual, typically to replace, correct or otherwise overcome a defect. The graft may comprise of cells that originate from the same individual; this graft is referred to herein by the interchangeable terms "autograft", "autologous transplant", "autologous implant" and "autologous graft".

A graft comprising cells from a genetically different individual of the same species is referred to herein by the following interchangeable terms: "allograft", "allogeneic transplant", "allogeneic implant" and "allogeneic graft". A graft from an individual to his identical twin is referred to herein as an "isograft", a "syngeneic transplant", a "syngeneic implant" or a "syngeneic graft". A "xenograft", "xenogeneic transplant" or "xenogeneic implant" refers to a graft from one individual to another of a different species.

Subjects to be treated by the present invention include both human and animal subjects, particularly mammalian subjects such as dogs, cats, horses, cattle, mice, monkeys, baboons, etc., for both human and veterinary medicine purposes and drug and device development purposes.

Nerves to be treated by the methods of the invention include afferent, efferent and mixed peripheral nerves such as somatic nerves, sensory-somatic nerves (including the cranial and spinal nerves), and autonomic nerves, which include sympathetic nerves, and parasympathetic nerves. Examples of nerves to be treated include, but are not limited to, cranial nerves, spinal nerves, nerves of the brachial plexus, nerves of the lumbar plexus, musculocutaneous nerve, femoral nerve, obturator nerve, sciatic nerve, the intercostal nerves, subcostal nerve, ulnar nerve, radial nerve, median nerve, pudendal nerve, saphenous nerve, common peroneal nerve, deep peroneal nerve, superficial peroneal nerve, and tibial nerve.

Damaged regions of nerves to be treated by the invention include those that have been subjected to a traumatic injury, such as crushed regions and severed (including fully and partially severed) regions in a limb, as well as nerves damaged in the course of a surgical procedure, e.g., as necessary to achieve another surgical goal. Damaged regions also include nerve regions that have degenerated due to a degenerative nerve disorder or the like, creating a "bottleneck" for axonal activity that can be identified by techniques such as electromyography and treated by use of the methods and devices of the present invention.

The types of peripheral injuries that are treatable according to the present invention are those in which a nerve has been damaged in which transection of the nerve may have occurred. The injury may be described as neurotmesis. The clinical definition of such injuries is also referred to under the "Sunderland System" as either fourth- degree or fifth-degree neurotmesis. In fourth-degree neurotmesis, there is interruption of all neural and supporting elements, the epineurium may be intact and the nerve is enlarged. In fifth-degree neurotmesis, there is complete transection with a loss of continuity of the nerve.

In a preferred embodiment, the nerve damage is peripheral nerve damage.

In yet another embodiment, the biomaterial has been obtained using plasma and/or stem cells which are autologous or allogeneic to the patient to be treated. Thus, in an embodiment, the plasma used to obtain the fibrin matrix is allogeneic with the subject which is treated with the neural graft according to the invention. In another embodiment, the stem cells which form part of the biomaterial are allogeneic with the subject which is treated with said graft according to the invention. In yet another embodiment, the plasma used to obtain the fibrin matrix and the stem cells which form part of the biomaterial are allogeneic with the subject which is treated with said graft according to the invention.

When used for the treatment of an injured nerve, the site of the severed nerve at which the biomaterial is to be inserted can be exposed using standard surgical techniques known in the art. Typically, the biomaterial is suitably floated onto the surgical field and connected to the proximal and/or nerve stump using microsurgical technique with microsurgical sutures (4-6 sutures 10/0) or using gluing technique with application of different types of tissue sealants or glues, or using staple technique, or a combination of the above. Once the biomaterial has been secured to both ends of the severed nerve structure, the surgical field can be closed using standard surgical techniques known to those skilled in the art.

Once implanted, the nerve graft is maintained under conditions in which nerve tissue is regenerated and/or nerve conduction (partial or complete) between the proximal nerve stump and the distal nerve stump is regained. A variety of methods for determining whether nerve regeneration has occurred are provided herein and these as well as other such methods are known to those of skill in the art. For example, clinical assessments such as a pin-prick (Siemionow, M, el al, Ann. Plast. Surg., 48:2 percent 1-285 (2002)) test, electrophysiological tests (e.g., somatosensory evoked potential evaluation (SSEP)), histomorphometric evaluations, nerve morphometry and morphology evaluations, Tinel sign, nerve conduction velocity, electromyography (EMG), muscle strength evaluation, etc. can be used {Surgery of the Peripheral Nerve, Susan E. MacKinnon and A. Lee Dellon, Thieme Medical Publishers, Inc., New York, 1988).

The biomaterial according to the invention may be used in the treatment of the following diseases which are usually with nerve damage:

- Peripheral Nerve Disorders: Peripheral nervous system disorders often result from damage to or dysfunction of the nerve cell body, myelin sheath, axons, or neuromuscular junction. Peripheral nervous system disorders can be genetic or acquired in conjunction with toxic, metabolic, traumatic, infectious, or inflammatory conditions. Peripheral neuropathies may affect one nerve (mononeuropathy), several discrete nerves (multiple mononeuropathy, or mononeuritis multiplex), or multiple nerves diffusely (polyneuropathy). Some peripheral nervous system disorders involve a plexus (plexopathy) or nerve root (radiculopathy) and may involve more than one site.
Because sensory and motor cell bodies have different compositions in different locations, a nerve cell body disorder typically affects either the sensory or motor component but rarely both. Damage to the myelin sheath, or demyelination, slows nerve conduction and affects predominantly heavily myelinated fibers. Demyelination causes sensory dysfunction (buzzing and tingling sensations), motor weakness, and diminished reflexes. Acquired demyelinating polyneuropathy often presents with profound motor weakness and minimal atrophy.
Peripheral nervous system disorders further include, but are not limited to, cervical spondylosis, disorders of neuromuscular transmission, Guillain-Barre syndrome (GBS), hereditary neuropathies, motor neuron disorders, myasthenia gravis, nerve root disorders, peripheral neuropathy, plexus disorders, spinal muscular atrophies, and thoracic outlet compression syndromes.

- Demyelinating Disorders, such as multiple sclerosis: Demyelination of peripheral nerve fibers may lead to slowing or failure of spike conduction, hyperexcitability, and interaction between nerve fibers. Slowed conduction occurs only at the site of demyelination, whereas the rest of the fiber has a normal conduction velocity. Unequal changes in conduction velocities in different fibers may contribute to altered sensation if the sensation requires the synchronous activity of many fibers in the nerve. Either spontaneous activity due to hyperexcitability or the cross-excitation of one demyelinated fiber by another could contribute to paresthesias, in which false peripheral events are signaled by inappropriately active nerves.

- Nerve Pain: Acute and chronic nerve pain may resul from injury and/or disease of pain receptors and nerve fibers and thus, can be managed or repair promoted with the implantable material of the present invention. Acute pain, which frequently occurs in response to tissue injury, results from activation of peripheral pain receptors (nociceptors) and their specific sensory nerve fibers (A delta fibers and C fibers). Chronic pain, which often relates to ongoing tissue injury, is thought to be caused by persistent activation of the peripheral pain receptor fibers. Chronic pain may also result from neuropathic pain, pain caused by damage or dysfunction of the peripheral or central nervous system. Nerve pain may also result from nerve edema, or the buildup of endoneurial fluid pressure following compression or injury to the nerve structure. Nerve edema often presents as local compressive effects and associated pain that persist following a compression or injury to the nerve. Nerve edema also can affect the nerve microenvironment, resulting in chronic pain or further nerve injury.

- Dorsal Root Disorders: Like peripheral nerves, dorsal roots can become injured or irritated due to compression, transection or disease and thus, are amenable for treatment with the biomaterial of the invention. Peripheral afferent fibers enter the spinal cord via the dorsal root ganglia, which are collections of pseudounipolar neurons lying just outside the spinal cord. The distal axons of dorsal root ganglion cells are peripheral nerve fibers. The proximal axons, called dorsal root fibers, enter the spinal cord. The effects of dorsal root injuries are generally similar to those produced by peripheral nerve injuries, but can differ in their severity and distribution. A common form of dorsal root injury is compression from herniation of an intervertebral disc. This type of injury can produce irritation of the dorsal root, resulting in pain over all or part of the affected mytome, sclerotome, or dermatome. Irritation can also produce paresthesias or hyperesthetic regions. If several dorsal roots receive sufficient damage, segmental anesthesia can result. Dorsal root injuries can also be caused by traction, inflammatory processes and diseases and ischemia.

- Spinal Cord Disorders: Spinal cord disorders can be managed and repair promoted with the biomaterial of the present invention. The majority of proximal axons from the dorsal root ganglia enter the dorsal horn of the spinal cord via the dorsal roots and contact secondary neurons within the same segment of the spinal cord, ascend or descend a few spinal segments before contacting secondary neurons and/or enter a major ascending spinal tract. Each of these dorsal root fibers has a characteristic distribution within a spinal cord segment. As dorsal root fibers enter the spinal cord, they divide roughly into a lateral aggregate of small, unmyelinated fibers and a more medial aggregate of larger myelinated fibers. This segmentation is the beginning of a sorting process that separates the fibers by size, and therefore by modality, since small fibers are largely nociceptive and thermoceptive, whereas the large fibers convey discriminative touch and proprioceptive information.

Further aspects [1] to [56] of the present invention include:

[1] A method for producing a nanostructured biomaterial capable of regenerating a nerve lesion comprising the steps of:

   (i) providing a hydrogel comprising a polysaccharide network, a fibrin network and cells embedded within said hydrogel and
   (ii) compacting the biomaterial of step (i) thereby expelling interstitial fluid from said hydrogel.

[2] A method according to [1] wherein the fibrin network is obtained by polymerizing a fibrinogen-comprising material.

[3] A method according to [2] wherein the fibrinogen-comprising material contains 1-5 g/L fibrinogen.

[4] A method according to [3] wherein the fibrinogen-comprising material is blood plasma.

[5] A method according to [2] or [3] wherein the polymerization of the fibrinogen-comprising material is carried out in the presence of a coagulation factor, a calcium source and, optionally, in the presence of an antifibrinolytic agent.

[6] A method as defined in [5] wherein the coagulation factor is thrombin.

[7] A method as defined in [5] or [6] wherein the antifibrinolytic agent is tranexamic acid.

[8] A method as defined in [7] wherein the calcium source is a calcium salt.

[9] A method as defined in [8] wherein the calcium salt is calcium chloride.

[10] A method according to any of [1] to [9] wherein the polysaccharide network is obtained by polymerizing a polysaccharide-comprising material.

[11] A method as defined in any of [1] to [10] wherein the polysaccharide is agarose.

[12] A method as defined in [11] wherein the agarose is low-melting point agarose.

[13] A method according to [12] wherein the concentration of agarose in the biomaterial before the compacting step is of about 0.025 to 0.3%, preferably 0.1%.

[14] A method according to any of [1] to [13] wherein the fibrin network is formed prior to the polysaccharide network.

[15] A method as defined in any of [1] to [14] wherein the cells are selected from the group consisting of stem cells, stromal cells, glial cells, neuronal cells or a combination thereof.

[16] A method as defined in [15] wherein the stem cells are mesenchymal stem cells.

[17] A method as defined in [16] wherein the mesenchymal stem cells are adipose tissue derived mesenchymal stem cells.

[18] A method as defined in any of [1] to [17] wherein when the fibrin network has been obtained from plasma, then the stem cells and the plasma are autologous.

[19] A method as defined in any of [1] to [18] wherein the density of stem cells in the biomaterial prior to the compacting step is of 10.000-15.000 cells/ml.

[20] A method according to any of [1] to [19] further comprising the step of incubating the biomaterial obtained in step (i) under conditions adequate for the proliferation of the stem cells.

[21] A method as defined in any of [1] to [20] wherein the compacting is a mechanical compaction of the biomaterial.

[22] A method as defined in [21] wherein the mechanical compaction is carried out by a method selected from the group consisting of applying a static weight, applying of a cam, applying one or more rollers, extrusion and centrifugation.

[23] A method according to [22] wherein an absorbent material is contacted with the biomaterial during the compacting step.

[24] A method according to [23] wherein a mechanical force is applied to increase contact between the absorbent material and the biomaterial.

[25] A method as defined in any of [1] to [24] wherein the compacting step is carried out until:

    (i) the compacted hydrogel shows a yield-stress value of about 5.8 Pa to 38.8 Pa,
    (ii) the compacted hydrogel shows a elastic modulus of 22 to 355 Pa and/or
    (iii) the compacting step is carried out until the compacted hydrogel shows a viscous modulus of 4 to 27 Pa or

[26] A method as defined in any of [1] to [24] wherein the compacting step is carried out until:

(i) the yield-stress of the biomaterial increases at least 6 times, preferably between 6 and 10 times,
(ii) elastic modulus of the biomaterial increases at least 2 times, preferably between 2 and 5 times and/or
(iii) the viscous modulus of the biomaterial increases at least 4 times, preferably between 4 and 5 times.

[27] A method as defined in any of [1] to [26] further comprising rolling up on itself a sheet of the biomaterial until at least one edge of the sheet of the biomaterial overlaps with the opposite edge.

[28] A method as defined in [27] wherein the rolling of the sheet is carried out to obtain a tubular structure of spiral section.

[29] A method as defined in [28] wherein the tubular structure substantially lacks any lumen.

[30] A method as defined in any of [26] to [29] further comprising ligating one or both of the ends of the tube.

[31] A biomaterial obtainable by a method according to any of [1] to [30].

[32] A biomaterial comprising a compacted nanostructured hydrogel wherein said hydrogel comprises a polysaccharide network, a fibrin network and cells embedded within said hydrogel.

[33] A biomaterial according to [32] wherein the compacted hydrogel shows a yield-stress value of about 5.8 Pa to 38.8 Pa, a elastic modulus of 22 to 355 Pa and/or a viscous modulus of 4 to 27 Pa.

[34] A biomaterial according to any of [31] to [33] wherein the polysaccharide-based hydrogel comprising fibrin and stem cells is obtained by polymerizing a fibrinogen-comprising material in the presence of the polysaccharide under conditions adequate for gelification of the polysaccharide.

[35] A biomaterial according to [34] wherein the fibrinogen-comprising material is blood plasma.

[36] A biomaterial according to [31] or [35] wherein the polymerization of the fibrinogen-comprising material is carried out in the presence of a coagulation factor, a calcium source and, optionally, in the presence of an antifibrinolytic agent.

[37] A biomaterial as defined in [36] wherein the coagulation factor is thrombin.

[38] A biomaterial as defined in [36] or [37] wherein the antifibrinolytic agent is tranexamic acid.

[39] A biomaterial as defined in any of [36] to [38] wherein the calcium source is a calcium salt.

[40] A biomaterial as defined in [39] wherein the calcium salt is calcium chloride.

[41] A biomaterial as defined in any of [31] to [40] wherein the polysaccharide is agarose.

[42] A biomaterial according to [41] wherein the concentration of agarose is between about 0.04% to about 0,5%, preferably about 0.16%.

[43] A biomaterial as defined in [41] or [42] wherein the agarose is low-melting point agarose.

[44] A biomaterial as defined in any of [32] to [43] wherein the cells are selected from the group consisting of stem cells, stromal cells, glial cells, neuronal cells and a combination thereof.

[45] A biomaterial as defined in [44] wherein the stem cells are mesenchymal stem cells.

[46] A biomaterial as defined in [45] wherein the mesenchymal stem cells are adipose tissue derived mesenchymal stem cells.

[47] A biomaterial as defined in [32] to [46] wherein the cells and the plasma are autologous.

[48] A biomaterial as defined in [31] to [47] wherein the density of cells in the biomaterial is of $1 \times 10^7$ to $1 \times 10^9$ cells per ml.

[49] A biomaterial as defined in any of [32] to [48] wherein the biomaterial has a tubular structure wherein one edge of a sheet of biomaterial overlaps with the opposite edge.

[50] A biomaterial as defined in [49] wherein the tubular structure has a spiral section.

[51] A method as defined in [49] or [50] wherein the tubular structure substantially lacks any lumen.

[52] A biomaterial as defined in any of [49] to [51] wherein one or both ends of the tubular structure are ligated.

[53] A biomaterial according to any of [31] to [52] for use in medicine.

[54] A biomaterial according to claim [31] to [52] for use in the treatment of a nerve damage.

[55] A biomaterial for use according to [54] wherein the nerve damage is peripheral nerve damage.

[56] A biomaterial for use according to [54] or [55] wherein the biomaterial has been obtained using plasma and/or stem cells which are autologous or allogeneic to the patient to be treated.

The invention will now be further described by way of reference to the following Examples and Figures which are provided for the purposes of illustration only and are not to be construed as being limiting on the invention.

**EXAMPLES**

**EXAMPLE 1: PREPARING A NANOSTRUCTURED ARTIFICIAL NERVE PRODUCT FOR REGENERATING THE PERIPHERAL NERVE IN AN ANIMAL MODEL (Ratus ratus LABORATORY RATS).**

**A.-Obtaining adipose tissue samples.**

For obtaining adipose tissue samples from laboratory rats, a surgical incision is made at the level of the inguinal area of the animals subjected to general anesthesia under asepsis conditions. A small adipose tissue biopsy is then extracted (ideally around 1 cm$^3$). The wound is then surgically repaired. The extracted sample is sent to the laboratory as soon as possible in sterility conditions.

**B.- Generating adipose tissue-derived mesenchymal stem cells (AT-MSC) cell cultures.**

When the sample reaches the laboratory it is washed several times with sterile saline, culture medium or phosphate buffer supplemented with antibiotics (500 U/ml of penicillin G and 500 kg/ml of streptomycin) and antimycotics (1.25 kg/ml of amphotericin B) to prevent possible contamination of the sample. The sample is then mechanically fragmented to obtain small explants prior to digestion. The tissue explants are then subjected to enzymatic digestion in 3% *Clostridium hystolyticum* type I collagenase solution for six to eight hours at 37°C under stirring. The isolated cells are finally collected by means of centrifugation and are cultured in DMEM culture medium supplemented with 10% fetal bovine serum and antibiotics (500 U/ml of penicillin G and 500 kg/ml of streptomycin) and antimycotics (1.25 kg/ml of amphotericin B) in Falcon culture flasks to generate primary cell cultures using standard culture medium (fetal bovine serum-enriched DMEM).

**C.- Constructing an artificial replacement for the nanostructured peripheral nerve by means of tissue engineering.**

1- Generating replacements for the peripheral nerve with adipose tissue-derived mesenchymal stem cells (AT-MSC) immersed in their thickness using fibrin-agarose extracellular matrices. To prepare 10 ml of nerve replacement:

- Obtain 7.6 ml of blood plasma (possibly autologous).
- Add 100,000-150,000 AT-MSC previously cultured and resuspended in 750 $\mu$l of DMEM culture medium.
- Add 150 $\mu$l of tranexamic acid at 100 mg/ml to prevent fibrin gel fibrinolysis.
- Add 1 ml of 1% ClCa$_2$ to induce the coagulation reaction and generate a fibrin fiber network.
- Rapidly add 0.5 ml of 2% type VII agarose dissolved in PBS and heated until reaching the melting point and gentle mixing by means of stirring. The final concentration of agarose in the mixture will be 0.1%. The agarose concentration range allowing viable nerve products ranges between and 0.025% and 0.3% and must be established in relation to the target patient of use.

- Aliquot in sterile Petri dishes as soon as possible.
- Leave to polymerize at rest for at least 30 minutes at 37°C.

2- Add culture medium on the surface of the generated tissue (the same medium that was used for the primary cell cultures) and maintain in an incubator at 37°C with 5% $CO_2$ in a humid atmosphere following standard cell culture protocols. These artificial tissues are maintained in culture between 1 and 7 days to favor the cell growth and proliferation (usually 3 days), the culture medium being refreshed every 3 days.

3- Extract the artificial tissue from the Petri dish in which it was generated and perform its nanostructuration. To that end, the tissue is deposited on a 3-5 mm thick sterile filter paper placed on a flat sterile glass surface. Place a fragment of sterile tulle netting, porous cloth, nylon or polycarbonate membrane or filter between the artificial tissue and the sterile paper to prevent top-most surface layer of the nerve replacement from adhering to the filter paper. Then place a second fragment of sterile tulle netting, porous cloth, nylon or polycarbonate membrane or filter on the artificial tissue and deposit another 3-5 mm thick sterile filter paper on it. Deposit a flat sterile glass fragment on the surface of the filter paper and place an object of approximately 250 g by weight on it (Figure 1). The entire process must be carried out in a laminar flow hood at room temperature for 10 minutes. The objective of this process is to significantly reduce the hydration levels of the product to reach optimal consistency and elasticity levels.
After this process, carefully extract the nanostructured tissue trying to prevent it from excessively dehydrating.

4- Generate a nanostructured three-dimensional tubular structure by means of rolling the material up. To that end, the nanostructured tissue is first cut to generate a quadrangular or rectangular structure, depending on the anatomical area in which it is subsequently going to be implanted and on the length of the nerve defect to be repaired. The tissue is then folded by means of rolling it up using a sterile thin pipette or spatula, taking care to not leave a lumen therein. The consistency and the high adherence levels of the tissue allow an easy rolling and maintenance. If necessary, a ligature can be applied to each end of the tube to maintain its structure.

## EXAMPLE 2: BIOMATERIAL USING SYNTHETIC COLLAGEN TUBES FILLED WITH FIBRIN-AGAROSE BIOMATERIALS FOR REGENERATING THE PERIPHERAL NERVE IN AN ANIMAL MODEL (*Ratus ratus* LABORATORY RATS).

1- Generating a fibrin-agarose hydrogel which is introduced into the lumen of a commercial synthetic collagen nerve guide (NeuraGen). To prepare 10 ml of hydrogel:

- Obtain 7.6 ml of blood plasma (possibly autologous),
- Add 750 $\mu$l of DMEM culture medium.
- Add 150 $\mu$l of tranexamic acid to prevent fibrin gel fibrinolysis.
- Add 1 ml of 1% $ClCa_2$ to induce the coagulation reaction and generate a fibrin fiber network.
- Rapidly add 0.5 ml of 2% type VII agarose dissolved in PBS and heated until reaching the melting point and gentle mixing by means of stirring. The final concentration of agarose in the mixture will be 0.1%. The agarose concentration range allowing viable nerve products ranges between 0.025% and 0.3% and must be established in relation to the target patient of use.
- Introduce the liquid mixture into the lumen of the synthetic collagen guides as soon as possible. To favor polymerization, the entire synthetic collagen guide is introduced into a liquid mixture of fibrin-agarose biomaterial in a Petri dish.
- Leave to polymerize at rest for at least 30 minutes at 37°C.

2- Extract the collagen guide filled with biomaterial from the Petri dish in which it was generated and implant it attempting to avoid excessive dehydration.

**EXAMPLE 3: BIOMATERIAL OBTAINED USING SYNTHETIC COLLAGEN TUBES FILLED WITH FIBRIN-AGAROSE BIOMATERIALS WITH ADIPOSE TISSUE-DERIVED STEM CELLS.**

**A.-Obtaining adipose tissue samples.**

For obtaining adipose tissue samples from laboratory rats, a surgical incision is made at the level of the inguinal area of the animals subjected to general anesthesia under asepsis conditions. A small adipose tissue biopsy is then extracted (ideally around 1 cm$^3$). The wound is then surgically repaired. The extracted sample is sent to the laboratory as soon as possible in sterility conditions.

**B.- Generating adipose tissue-derived mesenchymal stem cells (AT-MSC) cell cultures.**

When the sample reaches the laboratory it is washed several times with sterile saline, culture medium or phosphate buffer supplemented with antibiotics (500 U/ml of penicillin G and 500 kg/ml of streptomycin) and antimycotics (1.25 kg/ml of amphotericin B) to prevent possible contamination of the sample. The sample is then mechanically fragmented to obtain small explants prior to digestion. The tissue explants are then subjected to enzymatic digestion in 3% *Clostridium hystolyticum* type I collagenase solution for six to eight hours at 37°C under stirring. The isolated cells are finally collected by means of centrifugation and are cultured in DMEM culture medium supplemented with 10% fetal bovine serum and antibiotics (500 U/ml of penicillin G and 500 kg/ml of streptomycin) and antimycotics (1.25 kg/ml of amphotericin B) in Falcon culture flasks to generate primary cell cultures using standard culture medium (fetal bovine serum-enriched DMEM).

**C.- Constructing an artificial replacement for the nanostructured peripheral nerve by means of tissue engineering.**

Generating a fibrin-agarose hydrogel which is introduced into the lumen of a commercial synthetic collagen nerve guide (NeuraGen). To prepare 10 ml of hydrogel:

- Obtain 7.6 ml of blood plasma (possibly autologous).
- Add 100,000-150,000 AT-MSC previously cultured and resuspended in 750 μl of DMEM culture medium.
- Add 150 μl oftranexamic acid to prevent fibrin gel fibrinolysis.
- Add 1 ml of 1% CaCl$_2$ to induce the coagulation reaction and generate a fibrin fiber network.
- Rapidly add 0.5 ml of 2% type VII agarose dissolved in PBS and heated until reaching the melting point and gentle mixing by means of stirring. The final concentration of agarose in the mixture will be 0.1%. The agarose concentration range allowing viable nerve products ranges between 0.025% and 0.3% and must be established in relation to the target patient of use.
- Introduce the liquid mixture into the lumen of the synthetic collagen guides as soon as possible. To favor polymerization, the entire synthetic collagen guide is introduced into a liquid mixture of fibrin-agarose biomaterial in a Petri dish.
- Leave to polymerize at rest for at least 30 minutes at 37°C.

2- Add culture medium on the surface of the generated hydrogel (the same medium that was used for the primary cell cultures) and maintain in an incubator at 37°C with 5% CO$_2$ in a humid atmosphere following standard cell culture protocols. These artificial tissues are maintained in culture for about 24 h (range of 1 - 7 days) to favor cell growth and proliferation, the culture medium being refreshed every 3 days.

3- Extract the collagen guide filled with biomaterial and AT-MSC from the Petri dish in which it was generated and implant it attempting to avoid excessive dehydration.

**EXAMPLE 4: DETERMINATION OF THE VARIATIONS IN PHYSICOCHEMICAL PARAMETERS DURING THE COMPACTION STEP.**

The rheological properties of the bioengineered tissues were measured using a Bohlin CS10 controlled-stress rheometer (Bohlin Instruments, England) with a plate-plate measuring cell configuration. The plate diameter was 40 mm and the gap between the plates was, depending on the sample thickness, between 1 and 2 mm. All determinations were performed at 36.0 ± 0.1 °C. In order to avoid excessive drying of the samples, the artificial tissues were maintained in a vapor saturated atmosphere.

Two different experimental methods were followed: steady-state and dynamic measurements. For the steady-state method, a shear stress ($\sigma$) ramp from 0.06 to 20 Pa was applied to the samples and the corresponding

shear rate ($\dot{\gamma}$) was recorded. The time elapsed between two consecutive steps in the shear ramp was 3 s. The dynamic yield stress ($\sigma_y$), which corresponds to the minimum shear required to provoke a significant shear flow in the material, can be estimated by fitting the following equation:

$$\sigma = \sigma_y + \eta\dot{\gamma} \qquad (1)$$

to the experimental data recorded in the post-yield regime, in which a linear dependency between $\sigma$ and $\dot{\gamma}$ is apparent. The parameter $\eta$ is the so-called apparent viscosity.

In dynamic measurements (oscillatory measurements or simply oscillometry) a sinusoidal shear stress given by

$$\sigma = \sigma_0\, sin\,\omega t \qquad (2)$$

was applied to the sample, and the corresponding (oscillatory) shear strain

$$\gamma = \gamma_0\, sin\,(\omega t - \delta) \qquad (3)$$

was recorded. In equations (2) and (3), $\omega$ (rad/s) is the angular frequency, $\sigma_0$ and $\gamma_0$ are the stress and strain amplitudes, respectively. A lag ($\delta$) between the phase angles of the stress and strain exists, which is characteristic of each viscoelastic material. The shear stress applied depends on the frequency ($f = \omega 2\pi$, Hz) and the amplitude of the signal. For such a kind of experiments, we can obtain the so-called viscoelastic moduli, which completely characterize the viscoelasticity of the materials (Macosko, C.W., 1994, Rheology: Principles, Measurements and Applications, Wiley, New York ): the elastic or storage modulus ($G'$) and the viscous or loss modulus ($G''$).

Two different experiments can be performed: (i) amplitude sweep and (ii) frequency sweep. In amplitude sweeps, the frequency is maintained at a constant value (usually $f$ = 1 Hz) and the shear amplitude is increased within a range wide enough to reach the non-linear viscoelastic region. This region is characterized by a non-linear response of the viscoelastic moduli, for $\sigma_0$ values larger than certain critical shear amplitude ($\sigma_c$). For $\sigma_0$ values smaller than $\sigma_c$, a pseudoplateau is usually observed when $G'$ and $G''$ are plotted as a function of $\sigma_0$. Thus, in this region, usually called the viscoelastic linear region (VLR), the vicoelastic moduli do not depend on the shear amplitude.

Once the values of $\sigma_0$ pertaining to the VLR were determined, the second type of dynamic measurements (frequency sweep) was carried out. In these measurements, the amplitude of the oscillatory shear was maintained at a constant value well into the VLR, and a frequency sweep was carried out. As a result, the dependency of the viscoelastic moduli on the frequency was obtained, which represents the characteristic oscillogram of the sample. In the present work the frequency range studied was 0.01 - 20 Hz.

The yield stress, elastic modulus and viscous modulus of the biomaterial obtained as described in example 1 using different fibrin and agarose concentrations were determined as described above and compared with the values prior to the compacting step.

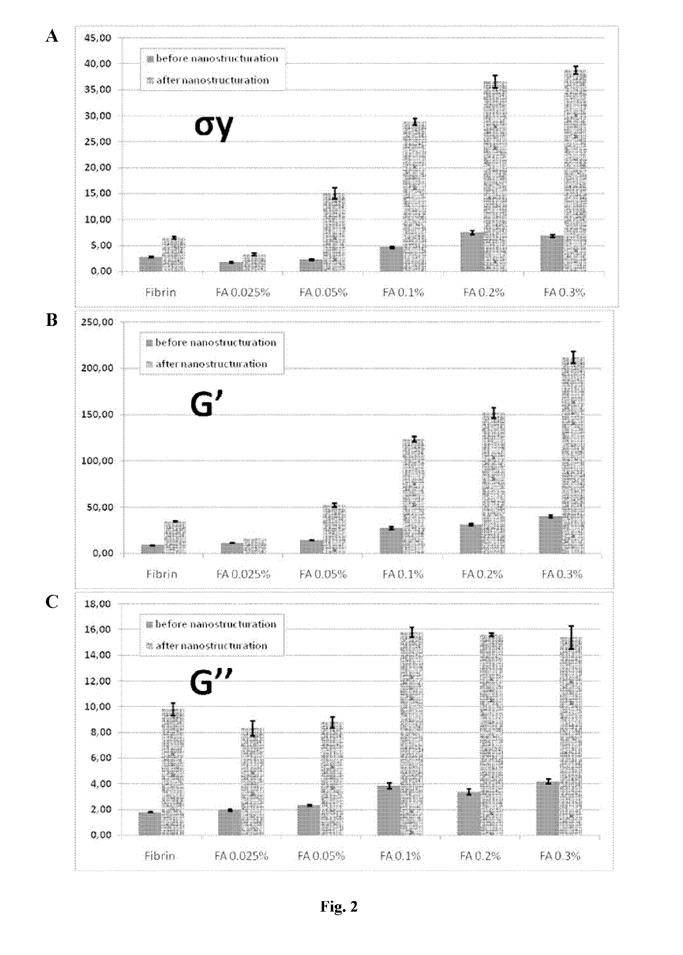

The compaction of the biomaterial results in an increase in yield stress with the agarose concentration (p=0.719, p<0.001) reaching values six to ten times higher than the ones of the non- compacted biomaterial (e.g., it varies from 5.8 Pa for the fibrin construct until 38.8 Pa for the fibrin with 0.3% agarose concentration) (Fig. 2A)

Moreover, the compacting resulted in a biomaterial having a elastic modulus of 22 to 355 Pa, compared to elastic modulus values of 9 to 80 Pa for the non-nanostructured biomaterial, depending in both cases on the agarose concentrations of the substitute (Fig. 2B).

The viscous modulus of the non-compacted biomaterial and of the compacted biomaterial ranged from 1 to 6 Pa and 4 to 27 Pa, respectively, depending on the agarose concentrations of the substitute (Fig. 2C).

## EXAMPLE 5: GENERATING A PERIPHERAL NERVE LESION MODEL AND EXPERIMENTAL GROUPS.

To evaluate the products generated by means of tissue engineering shown in Examples 1, 2 and 3, the products were implanted in laboratory animals (laboratory rats) in which irreversible damage to the peripheral nerve was induced as described below.

Once the animals were anesthetized with ketamine and acepromazine, a lateral approach in the right femur was performed in each animal, the intermuscular septum existing between the biceps and quadriceps being sectioned, exposing the femur and, right below it, the sciatic nerve. A defect is then generated in the proximal sciatic nerve at the branch of the gastrocnemius muscle group, approximately one centimeter from the exit of the nerve through the intervertebral foramen coinciding in most cases with the mid-third of the diaphysis of the femur. In all cases except in the positive controls, a 10 mm fragment of the left sciatic nerve was extirpated (this type of defect cannot spontaneously be repaired).

Six experimental groups were established in this project as described below.

• Group 0. Animals not subjected to any surgical intervention (POSITIVE CONTROL).

• Group 1. Lesion of the sciatic nerve with a one centimeter nerve defect without surgical repair thereof (NEGATIVE CONTROL).

• Group 2. Lesion of the sciatic nerve with a one centimeter nerve defect and microsurgical repair of both ends of the nerve by means of a nerve gluide formed by a collagen tube filled with heparinized saline sutured at both ends of the defect. It is important to point out that this is the surgical technique of choice for the clinical repair of peripheral nerve defects when autologous nerve grafts are not available.

• Group 3. Lesion of the sciatic nerve with a one centimeter nerve defect and microsurgical repair of both ends of the nerve by means of a nerve gluide formed by a commercial collagen tube filled with fibrin-agarose biomaterials sutured at both ends of the defect.

• Group 4. Lesion of the sciatic nerve with a one centimeter nerve defect and microsurgical repair of both ends of the nerve by means of a nerve guide formed by a commercial collagen tube filled with autologous adipose tissue-derived mesenchymal stem cells (AT-MSC) immersed in fibrin-agarose biomaterials sutured at both ends of the defect.

• Group 5. Lesion of the sciatic nerve with nerve defect and microsurgical repair of the nerve defect by means of implanting the artificial nerve product described in Example 1 of this specification (nanostructured artificial nerve product) between the proximal end and the distal end the lesioned nerve.

In Group 2, immediately after the surgical section of the nerve and extirpation of a fragment thereof, a commercial neural collagen gluide for clinical use, NeuraGen®, was implanted and sutured at both ends of the sectioned sciatic nerve using 10-0 monofilament sutures, joining the epineuron of the nerve with the neural guide and introducing approximately 2 mm of the sectioned nerve end into the guide. The same technique was used in Group 3, implanting a commercial collagen nerve guide filled with fibrin-agarose biomaterials as described in Example 2. A commercial collagen nerve guide filled with fibrin-agarose biomaterials and AT-MSC as described in Example 3 was used in Group 4. The replacement or nerve guide generated by means of nanotechnology was directly implanted in Group 5 without any commercial collagen nerve guides.

Only the left sciatic nerve was operated on in each animal in Groups 1 to 5, leaving the right sciatic nerve intact as the control. In all cases, the results obtained in each group of animals were evaluated 3 months after the intervention.

**EXAMPLE 6: EVALUATING THE PRODUCTS GENERATED IN AN ANIMAL MODEL OF REGENERATING THE PERIPHERAL NERVE. CLINICAL PARAMETERS**

Once the follow-up period ended, the presence of neurotrophic ulcers and amputations or self-mutilations (signs of chronic pain, numbness, stiffness, or other nerve pathologies) which appeared in the limb of every animal that was operated on were first studied in all the animals. The results show that all the animals of Group 1 (negative control) presented neurotrophic ulcers, and 50% of them further presented some type of self-mutilation. The animals of Group 2 (commercial collagen guide) presented a 58% incidence of neurotrophic ulcers and a 17% incidence of self-mutilations. When stem cells were added to the biomaterials used in combination with the commercial collagen tubes (Group 4), the incidence of neurotrophic ulcers dropped to 36% and the incidence of self-mutilations dropped to 9%. Finally, the animals of Group 5 (nanostructured artificial nerve product) did not present any case of neurotrophic ulcers or self-mutilations, as occurred with the animals of Group 0 (positive control), which suggests that the application of biomaterials or of nanostructured nerve replacements could protect against the onset of neurotrophic ulcers (Figure 2).

Then the functional motor recovery state was evaluated by means of studying the **Spontaneous Motor Activity** (movements, slips and slow and fast elevations in every animal and in the three dimensions of space) (Ferrer *et al.,* 1987). The results show that the animals of the negative control (Group 1) presented a considerable reduction of spontaneous motor activity determined by slow and fast movements. Secondly, the rats of Group 2 showed spontaneous motor activity values similar to those described for Group 1, so the application of commercial collagen tubes did not improve spontaneous motor activity. With respect to Groups 3 and 4, the animals showed a considerable increase in the number of recorded movements, which suggests that the application of fibrin-agarose biomaterials with or without cells in the lumen of the collagen tube could improve these parameters. Finally, the animals subjected to repair using nanostructured artificial nerve products (group 5) showed spontaneous motor activity similar to that of the positive controls (group 0), there being no significant differences.

Finally, the **length of the footprint** determined by the distance between the footpad and the longest toe of the same extremity measured in the bearing area of said extremity was evaluated. The results (Figure 3) show that the animals of Group 1 suffer a significant neurogenic retraction of the intrinsic musculature of the paw, reducing the length of the footprint. When commercial tubes are used for nerve repair (Group 2), a clear increase in the length of the print can be seen, which does not vary in Group 3. In Group 4, however, an increase in the length of the print with respect to Groups 2 and 3 is observed. Finally, there are no differences between the positive control group and the group subjected to repair using nanostructured artificial nerve products (Group 5) (mean 37 and 38 mm, respectively), this distance being significantly greater than the one found in the negative controls (mean 3 mm). These results suggest that the animals of the negative control group present a neurogenic retraction of the intrinsic musculature of the extremity, which does not occur in the positive controls or in Group 5, being mild in Group 4.

## EXAMPLE 7: EVALUATING THE PRODUCTS GENERATED IN AN ANIMAL MODEL OF REGENERATING THE PERIPHERAL NERVE. ELECTROMYOGRAPHIC CLINICAL PARAMETERS

In another approach to determine the degree of nerve regeneration, all the animals were subjected to neurophysiological study, being the results shown in Table 1.

| | | Denervation | | | | Reinnervation | | | |
|---|---|---|---|---|---|---|---|---|---|
| | | BICEPS | GAST. | ANTTIB | EDB | BICEPS | GAST. | ANTTIB | EDB |
| Group | 1 | 2.3 | 3.0 | 3.0 | 3.0 | 0.3 | 0.0 | 0.0 | 0.0 |
| | 2 | 1.6 | 1.9 | 2.3 | 2.9 | 0.9 | 0.6 | 0.0 | 0.0 |
| | 3 | 1.0 | 1.2 | 2.1 | 2.9 | 1.9 | 1.4 | 0.4 | 0.0 |
| | 4 | 0.0 | 0.8 | 1.9 | 2.6 | 2.0 | 1.3 | 0.4 | 0.1 |
| | 5 | 0.0 | 0.6 | 1.6 | 2.5 | 2.6 | 2.2 | 1.3 | 0.5 |

Table 1. Neurophysiological study conducted in the animals of Groups 1 to 5. The signs of denervation and the signs of reinnervation were analyzed for each muscle group (biceps femoralis, gastrocnemius (GAST.), tibialis anterior (ANTTIB), extensor digitorum brevis (EDB)) on a scale ranging between 0 (absence), 1 (mild presence), 2 (moderate presence) and 3 (intense presence).

The results obtained in Group 1 (negative control) show the existence of electromyographic signs of severe denervation, no signs of reinnervation being observed in any muscle group except in the femoral biceps which, probably due to its proximity to the proximal end of the lesioned nerve shows isolated phenomena of reinnervation possibly due to the penetration of axonal sprouting into the muscle fibers.

However, in the remaining muscle groups more distal to the lesion there is no presence of reinnervation in any case.

In Group 2 (a commercial collagen tube implant), it can be observed how the degree of denervation is lower than that group that does not receive any type of treatment of the lesioned nerve (Group 1) but overall, it continues to be rather high. Concerning reinnervation, there is a complete absence of reinnervation of the anterior tibia muscles and foot muscles probably due to the large distance to the muscle fibers and the anatomical difficulty for the nerve to reach them.

In contrast, the use of biomaterials in the lumen of the commercial tube (Group 3) resulted in an improvement of the degree of denervation of the studied muscles, fundamentally at the calf muscle level where there are significant differences with respect to the group treated simply with commercial collagen guides (Group 2). Greater reinnervation of the biceps and calves than in Group 2 is in turn observed, there being no differences for the more distal muscles.

When AT-MSC are added to the biomaterial (Group 4), the degree of denervation of all the muscles generally decreases, highlighting the absence of signs of denervation in the femoral biceps in all the animals of this group.

Furthermore, the degree of reinnervation of all the muscle groups very slightly increases with respect to the animals treated with biomaterials in an isolated manner (Group 3), there being no significant differences for any of the reinnervation variables. However, signs of slight reinnervation at the level of the extensor digitorum brevis muscle were detected in an animal of this group.

Finally, in the animals of Group 5 (nanostructured artificial nerve product implant), the degree of denervation substantially decreases with respect to the previous groups, except for the extensor digitorum brevis muscle. With respect to the reinnervation of the muscles distal to the lesion, signs indicating the existence of intense reinnervation phenomena are seen in all the studied muscle groups, being mild for the extensor digitorum brevis muscle.

**Claims**

1. A method for producing a nanostructured biomaterial capable of regenerating a nerve lesion comprising the steps of:

    (i) providing a hydrogel comprising a polysaccharide network, a fibrin network and cells embedded within said hydrogel and
    (ii) compacting the biomaterial of step (i) thereby expelling interstitial fluid from said hydrogel.

2. A method as defined in claim 1 wherein the cells are selected from the group consisting of stem cells, stromal cells, glial cells, neuronal cells or a combination thereof.

3. A method as defined in claims 1 or 2 wherein the compaction is carried out by a method selected from the group consisting of applying a static weight, applying of a cam, applying one or more rollers, extrusion and centrifugation.

4. A method as defined in any of claims 1 to 3 wherein the compacting step is carried out until:

    (i) the compacted hydrogel shows a yield-stress value of about 5.8 Pa to 38.8 Pa,
    (ii) the compacted hydrogel shows a elastic modulus of 22 to 355 Pa and/or
    (iii) the compacting step is carried out until the compacted hydrogel shows a viscous modulus of 4 to 27 Pa or

5. A method as defined in any of claims 1 to 4 wherein the compacting step is carried out until:

    (i) the yield-stress of the biomaterial increases at least 6 times, preferably between 6 and 10 times,
    (ii) the elastic modulus of the biomaterial increases at least 2 times, preferably between 2 and 5 times and/or
    (iii) the viscous modulus of the biomaterial increases at least 4 times, preferably between 4 and 5 times.

6. A method as defined in any of claims 1 to 5 further comprising rolling up on itself a sheet of the biomaterial until at least one edge of the sheet of the biomaterial overlaps with the opposite edge.

7. A method as defined in claim 6 wherein the rolling of the sheet is carried out to obtain a tubular structure of spiral section.

8. A biomaterial obtainable by a method according to any of claims 1 to 7.

9. A biomaterial comprising a compacted nanostructured hydrogel wherein said hydrogel comprises a polysaccharide network, a fibrin network and cells embedded within said hydrogel.

10. A biomaterial according to claim 9 wherein the compacted hydrogel shows a yield-stress value of about 5.8 Pa to 38.8 Pa, a elastic modulus of 22 to 355 Pa and/or a viscous modulus of 4 to 27 Pa.

11. A biomaterial as defined in claims 9 or 10 wherein the cells are selected from the group consisting of stem cells, stromal cells, glial cells, neuronal cells and a combination thereof.

12. A biomaterial as defined in any of claims 9 to 11 wherein the biomaterial has a tubular structure wherein one edge of a sheet of biomaterial overlaps with the opposite edge.

13. A biomaterial as defined in claim 12 wherein the tubular structure has a spiral section.

**14.** A biomaterial according to any of claims 8 to 13 for use in medicine.

**15.** A biomaterial according to claims 8 to 13 for use in the treatment of a nerve damage.

GLASS
FILTER PAPER
NYLON CLOTH
ARTIFICIAL TISSUE
FILTER PAPER
GLASS

**Fig. 1**

**Fig. 2**

Fig. 3

Fig. 4

Europäisches
Patentamt
European
Patent Office
Office européen
des brevets

# EUROPEAN SEARCH REPORT

Application Number

EP 11 38 2349

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/023843 A2 (SERVICIO ANDALUZ DE SALUD [ES]; UNIV GRANADA [ES]; ALAMINOS MINGORANCE) 3 March 2011 (2011-03-03) | 1-14 | INV.<br>A61L27/20<br>A61L27/22 |
| Y | * page 6, line 27 - page 7, line 21 *<br>* page 10, line 16 - page 11, line 2 *<br>* page 24, line 28 - page 27, line 2 *<br>* page 28, line 14 - page 33, line 17 *<br>* page 48, line 6 - page 49, line 25 *<br>* example 8 *<br>* figures 1, 8-10 *<br>* claims 1-40 * | 15 | A61L27/26<br>A61L27/38<br>A61L27/52 |
| Y | NIETO-AGUILAR R ET AL: "Pluripotential differentiation capability of human adipose-derived stem cells in a novel fibrin-agarose scaffold",<br>JOURNAL OF BIOMATERIALS APPLICATIONS, TECHNOMIC, LANCASTER, PA, US,<br>vol. 25, no. 7, 1 March 2011 (2011-03-01), pages 743-768, XP008151083,<br>ISSN: 0885-3282, DOI:<br>10.1177/0885328209360425<br>[retrieved on 2010-03-17]<br>* abstract *<br>* page 745, paragraph 1 *<br>* page 746, paragraph 2 *<br>* page 755, paragraph 2 - page 756, paragraph 2 *<br>* page 763, paragraph 1 *<br>* page 764, paragraph 2 *<br>* page 765, paragraph 1 * | 15 | |

TECHNICAL FIELDS
SEARCHED (IPC)

A61L

The present search report has been drawn up for all claims

| Place of search | Date of completion of the search | Examiner |
|---|---|---|
| The Hague | 26 April 2012 | López García, Mónica |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 11 38 2349

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2012

| Patent document cited in search report | Publication date | Patent family member(s) | Publication date |
|---|---|---|---|
| WO 2011023843 A2 | 03-03-2011 | NONE | |

For more details about this annex : see Official Journal of the European Patent Office, No. 12/82

EPO FORM P0459

**EP 2 594 295 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4863668 A
- WO 0154593 A
- US 6511958 B
- US 6436704 B
- US 6280718 B
- US 6258597 B
- US 6184035 B
- US 6132708 A
- US 58375739 B
- US 6506727 B, Hansson
- US 6890531 B, Horie
- US 5843914 A, Johnson, Jr.
- US 5488099 A, Persson
- US 5438121 A, Barde
- US 5235043 A, Collins
- US 6005081 A, Burton

### Non-patent literature cited in the description

- **TONG, X. et al.** *Brain Research,* 1994, vol. 663, 155-162
- **GARRIDO-GÓMEZ et al.** *Histology and Histopathology,* 2011, vol. 26 (1), 50.4.o4
- **TOHILL M. et al.** *Biotechnol. Appl. Biochem.,* 2004, vol. 40, 17-24
- **KOKAI L.E. et al.** *Plast Reconstr Surg,* 2005, vol. 1 16 (5), 1453-60
- **CHEN Y. et al.** *Cell Mol Life Sci,* 2006, vol. 63 (14), 1649-57
- **HAUNER et al.** *J. Clin. Invest.,* 1989, vol. 84 (5), 1663-1670
- **HAUNER et al.** *Horm. Metab. Res.,* 1988, vol. 19, 35-39
- **AUST et al.** *Cytotherapy,* 2004, vol. 6 (1), 7-14
- **HALVORSEN et al.** *Metabolism,* 2001, vol. 50, 407-413
- **MITCHELL et al.** *Stem Cells,* 12 January 2006, 2005-0235
- **SIEMIONOW, M.** *Ann. Plast. Surg.,* 2002, vol. 48 (2), 1-285
- **SUSAN E. MACKINNON ; A. LEE DELLON.** Surgery of the Peripheral Nerve. Thieme Medical Publishers, Inc, 1988
- **MACOSKO, C.W.** Rheology: Principles, Measurements and Applications. Wiley, 1994

33